(19) Europäisches Patentamt
European Patent Office
Office européen des brevets

(11) EP 2 263 704 A1

(12) EUROPÄISCHE PATENTANMELDUNG

(43) Veröffentlichungstag:
**22.12.2010 Patentblatt 2010/51**

(51) Int Cl.:
***A61L 15/60*** *(2006.01)* ***A61L 15/24*** *(2006.01)*
***A61L 15/18*** *(2006.01)*

(21) Anmeldenummer: **10173417.6**

(22) Anmeldetag: **14.10.2005**

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **20.10.2004 DE 102004051242**

(62) Dokumentnummer(n) der früheren Anmeldung(en) nach Art. 76 EPÜ:
**05798199.5 / 1 804 842**

(27) Früher eingereichte Anmeldung:
**14.10.2005 PCT/EP2005/011073**

(71) Anmelder: **BASF SE**
**67056 Ludwigshafen (DE)**

(72) Erfinder:
- **Riegel, Ulrich**
  **66849, Landstuhl (DE)**
- **Daniel, Thomas**
  **67165, Waldsee (DE)**
- **Hermeling, Dieter**
  **67459, Böhl-Iggelheim (DE)**
- **Elliott, Mark**
  **67063, Ludwigshafen (DE)**

Bemerkungen:
Diese Anmeldung ist am 19-08-2010 als Teilanmeldung zu der unter INID-Code 62 erwähnten Anmeldung eingereicht worden.

(54) **Feinteilige wasserabsorbierende Polymerpartikel mit hoher Flüssigkeitstransport- und Absorptionsleistung**

(57) Die Erfindung betrifft feinteilige wasserabsorbierende Polymerpartikel mit hoher Flüssigkeitstransport- und Absorptionsleistung, wobei die Zentrifugenretentionskapazität (CRC) mindestens 26 g/g, die Absorption unter einem Druck von 4,83 kPa (AUL0.7psi) mindestens 23 g/g und der Transportwert (TW) mindestens 15.000 $cm^3s$ beträgt, wobei der Transportwert (TW) das Produkt aus Flüssigkeitsweiterleitung (SFC) und Dochtabsorption nach 60 Minuten ($DA_{60}$) multipliziert mit $10^7$ ist, und wobei die Dochtabsorption nach 60 Minuten ($DA_{60}$) die Gewichtsmenge an 0,9 gew.-%iger Kochsalzlösung ist, die 70 g der wasserabsorbierenden Polymerpartikel in 60 Minuten aufnehmen, wobei sich die wasserabsorbierenden Polymerpartikel während der Messung in einem kreisrunden Gefäß mit einem Innendurchmesser von 6 cm befinden, welches unten mit einen Siebboden der Maschenweite 36 μm verschlossen ist, und der Siebboden drucklos mit 0,9 gew.-%iger Kochsalzlösung in Kontakt steht, Verfahren zu ihrer Herstellung sowie die Verwendung in Hygieneartikeln und Verpackungmaterialien.

EP 2 263 704 A1

## Beschreibung

Die vorliegende Erfindung betrifft feinteilige wasserabsorbierende Polymerpartikel mit hoher Flüssigkeitstransport- und Absorptionsleistung, Verfahren zu ihrer Herstellung sowie die Verwendung in Hygieneartikeln und Verpakkungsmaterialien.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils angegebenen Kombination, sondern auch in anderen Kombinationen verwendbar sind, ohne den Rahmen der Erfindung zu verlassen.

Wasserabsorbierende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate. Solche Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Zur Verbesserung der Anwendungseigenschaften, wie beispielsweise Flüssigkeitsleitfähigkeit (SFC) in der Windel und Absorption unter Druck (AUL), werden wasserabsorbierende Polymerpartikel im allgemeinen nachvernetzt. Diese Nachvernetzung kann in wässriger Gelphase durchgeführt werden. Vorzugsweise werden aber gemahlene und abgesiebte Polymerpartikel (Grundpolymer) an der Oberfläche mit einem Nachvernetzer beschichtet, getrocknet und thermisch nachvernetzt. Dazu geeignete Vernetzer sind Verbindungen, die mindestens zwei Gruppen enthalten, die mit den Carboxylatgruppen des hydrophilen Polymeren kovalente Bindungen bilden können.

US 5,599,335 offenbart, dass gröbere Partikel eine höhere Flüssigkeitsleitfähigkeit (SFC) der gequollenen Gelschicht aufweisen. Weiterhin wird gelehrt, das die Flüssigkeitsleitfähigkeit (SFC) durch Nachvernetzung gesteigert werden kann, wobei aber immer die Zentrifugenretentionskapazität (CRC) und damit das Absorptionsvermögen der wasserabsorbierenden Polymerpartikel sinkt.

Dem Fachmann ist allgemein bekannt, dass durch Erhöhung der internen Vernetzung (mehr Vernetzer im Grundpolymer) als auch durch stärkere Nachvernetzung (mehr Nachvernetzer) die Flüssigkeitsleitfähigkeit (SFC) zu Lasten der Zentrifugenretentionskapazität (CRC) gesteigert werden kann.

In WO 04/069293 werden wasserabsorbierende Polymerpartikel offenbart, die mit wasserlöslichen Salzen polyvalenter Kationen beschichtet sind. Die Polymerpartikel weisen eine verbesserte Flüssigkeitsleitfähigkeit (SFC) und verbesserte Absorptionseigenschaften auf.

WO 04/069404 offenbart salzresistente wasserabsorbierende Polymerpartikel, die jeweils ähnliche Werte für die Absorption unter Druck (AUL) und die Zentrifugenretentionskapazität (CRC) aufweisen.

WO 04/069915 beschreibt ein Verfahren zur Herstellung wasserabsorbierender Polymerpartikel mit hoher Flüssigkeitsleitfähigkeit (SFC), die gleichzeitig über starke Kapillarkräfte verfügen, d.h. die wässrige Flüssigkeiten gegen die Schwerkraft aufsaugen können. Die Kapillarwirkung der Polymerpartikel wird durch eine spezielle Oberflächenbeschaffenheit erreicht. Dazu werden Partikel mit einer Größe von weniger als 180 μm aus dem Grundpolymer ausgesiebt, agglomeriert und mit den vorher abgetrennten Partikeln größer 180 μm vereinigt.

Für ultradünne Hygieneartikel werden feinteilige wasserabsorbierende Polymerpartikel ohne grobe Körner (Partikel) benötigt, da diese fühlbar wären und beim Verbraucher auf Ablehnung stoßen.

Die Flüssigkeitsweiterleitung (SFC) ist aber umso geringer, je kleiner die Partikel sind. Andererseits verfügen kleine Polymerpartikel auch über kleinere Poren, die den Flüssigkeitstransport durch Dochtabsorption innerhalb der Gelschicht verbessern.

In ultradünnen Hygieneartikeln spielt dies eine wichtige Rolle, da diese Konstruktionselemente enthalten können, welche zu 50 bis 100 Gew.-% aus wasserabsorbierenden Polymerpartikeln bestehen, so dass die Polymerpartikel im Gebrauch sowohl die Speicherfunktion für die Flüssigkeit übernehmen als auch den aktiven (Dochtabsorption) und passiven Flüssigkeitstransport (Flüssigkeitsweiterleitung). Je mehr Zellstoff durch wasserabsorbierende Polymerpartikel oder Synthesefasern ersetzt wird, desto mehr Transportfunktionen müssen die wasserabsorbierenden Polymerpartikel zusätzlich zu ihrer Speicherfunktion erfüllen.

Es bestand daher die Aufgabe wasserabsorbierende Polymerpartikel mit hoher Zentrifugenretentionskapazität (CRC), hoher Absorption unter Druck (AUL), hohem aktiven (Dochtabsorption) und passiven Flüssigkeitstransport (Flüssigkeitsweiterleitung) bereitzustellen, wobei die wasserabsorbierenden Polymere insbesondere eine hohe Flüssigkeitsweiterleitung (SFC) aufweisen sollten.

Eine weitere Aufgabe war die Bereitstellung optimierter wasserabsorbierender Polymerpartikel mit einem niedrigen mittleren Teilchendurchmesser.

Es bestand weiterhin die Aufgabe ein Verfahren zur Herstellung wasserabsorbierender Polymerpartikel mit hoher Zentrifugenretentionskapazität (CRC), hoher Absorption unter Druck (AUL), hohem aktiven (Dochtabsorption) und passiven Flüssigkeitstransport (Flüssigkeitsweiterleitung) bereitzustellen, wobei die wasserabsorbierenden Polymere insbesondere eine hohe Flüssigkeitsweiterleitung (SFC) aufweisen sollten.

Eine weitere Aufgabe war die Bereitstellung eines Verfahrens zur Herstellung wasserabsorbierender Polymerpartikel, wobei weiße Polymerpartikel erzeugt werden, die frei von wahrnehmbaren Gerüchen sind, insbesondere bei Flüssigkeitsbeladung.

Die Aufgabe wird gelöst durch Bereitstellung wasserabsorbierender Polymerpartikel, enthaltend

a) mindestens ein einpolymerisiertes ethylenisch ungesättigtes, säuregruppentragendes Monomer,

b) mindestens einen einpolymerisierten Vernetzer,

c) gegebenenfalls ein oder mehrere mit a) copolymerisierbare einpolymerisierte ethylenisch und/oder allylisch ungesättigte Monomere,

d) gegebenenfalls ein oder mehrere wasserlösliche Polymere, auf die die Monomere a), b) und ggf. c) zumindest teilweise aufgepfropft sind, und

e) mindestens einen umgesetzten Nachvernetzer,

wobei die Zentrifugenretentionskapazität (CRC) mindestens 26 g/g, die Absorption unter einem Druck von 4,83 kPa (AUL0.7psi) mindestens 23 g/g und der Transportwert (TW) mindestens 15.000 $cm^3s$ beträgt,
wobei der Transportwert (TW) das Produkt aus Flüssigkeitsweiterleitung (SFC) und Dochtabsorption nach 60 Minuten ($DA_{60}$) multipliziert mit $10^7$ ist, und
wobei die Dochtabsorption nach 60 Minuten ($DA_{60}$) die Gewichtsmenge an 0,9 gew.-%iger Kochsalzlösung ist, die 70 g der wasserabsorbierenden Polymerpartikel in 60 Minuten aufnehmen, wobei sich die wasserabsorbierenden Polymerpartikel während der Messung in einem kreisrunden Gefäß mit einem Innendurchmesser von 6 cm befinden, welches unten mit einen Siebboden der Maschenweite 36 μm verschlossen ist, und der Siebboden drucklos mit 0,9 gew.-%iger Kochsalzlösung in Kontakt steht.

Die Zentrifugenretentionskapazität wird gemäß der von der EDANA (European Disposables and Nonwovens Association) empfohlenen Testmethode Nr. 441.2-02 "Centrifuge retention capacity" bestimmt.

Die Absorption unter Druck wird gemäß der von der EDANA (European Disposables and Nonwovens Association) empfohlenen Testmethode Nr. 442.2-02 " Absorption under pressure" bestimmt.

Der Transportwert (TW) beträgt vorzugsweise mindestens 17.500 $cm^3s$, bevorzugt mindestens 20.000 $cm^3s$, besonders bevorzugt mindestens 22.500 $cm^3s$, ganz besonders bevorzugt mindestens 25.000 $cm^3s$, und üblicherweise nicht über 100.000 $cm^3s$.

Vorzugsweise weisen weniger als 2 Gew.-%, besonders bevorzugt weniger als 1,5 Gew.-%, ganz besonders bevorzugt weniger als 1 Gew.-%, der Polymerpartikel eine Partikelgröße von weniger als 150 μm auf. Die Partikelgröße wird gemäß der von der EDANA (European Disposables and Nonwovens Association) empfohlenen Testmethode Nr. 420.2-02 "Particle size distribution" bestimmt.

Die Säuregruppen des einpolymerisierten Monomeren a) sind vorzugsweise zu größer 60 mol-%, bevorzugt zu größer 61 mol-%, besonders bevorzugt zu größer 62 mol%, ganz besonders bevorzugt zu größer 63 mol%, und vorzugsweise zu höchstens 70 mol-%, bevorzugt zu höchstens 69 mol-%, besonders bevorzugt zu höchstens 68 mol-%, ganz besonders bevorzugt zu höchstens 67 mol-%, neutralisiert.

Geeignete Monomere für die einpolymerisierten Monomere a), b) und c) sind die unten beschriebenen Monomere i), ii) und iii).

Geeignete wasserlösliche Polymere für die zumindest teilweise gepfropften wasserlöslichen Polymere d) sind die unten beschriebenen wasserlöslichen Polymere iv).

Geeignete Nachvernetzer für die umgesetzten Nachvernetzer e) sind die unten beschriebenen Nachvernetzer v).

Der Wassergehalt der erfindungsgemäßen wasserabsorbierenden Polymerpartikel beträgt vorzugsweise weniger als 6 Gew.-%, besonders bevorzugt weniger als 4 Gew.-%, ganz besonders bevorzugt weniger als 3 Gew.-%.

Bevorzugte erfindungsgemäße wasserabsorbierende Polymerpartikel sind Polymerpartikel A, B und C mit den obengenannten Eigenschaften.

Polymerpartikel A:

Vorzugsweise weisen weniger als 2 Gew.-%, besonders bevorzugt weniger als 1,5 Gew.-%, ganz besonders bevorzugt weniger als 1 Gew.-%, der Polymerpartikel A eine Partikelgröße von über 600 μm auf.

Vorzugsweise weisen mindestens 90 Gew.-%, bevorzugt mindestens 95 Gew.-%, besonders bevorzugt mindestens 98 Gew.-%, ganz besonders bevorzugt mindestens 99 Gew.-%, der Polymerpartikel A eine Partikelgröße von 150 bis 600 μm auf.

Vorzugsweise weisen mindestens 70 Gew.-%, bevorzugt mindestens 80 Gew.-%, besonders bevorzugt mindestens 85 Gew.-%, ganz besonders bevorzugt mindestens 90 Gew.-%, der Polymerpartikel A eine Partikelgröße von 300 bis 600 μm auf.

Die Zentrifugenretentionskapazität (CRC) der Polymerpartikel A beträgt üblicherweise mindestens 26 g/g, vorzugsweise mindestens 27 g/g, bevorzugt mindestens 28 g/g, besonders bevorzugt mindestens 29 g/g, ganz besonders bevorzugt mindestens 30 g/g, und üblicherweise nicht über 50 g/g.

Die Absorption unter einem Druck von 4,83 kPa (AUL0.7psi) der Polymerpartikel A beträgt üblicherweise mindestens 23 g/g, vorzugsweise mindestens 24 g/g, bevorzugt mindestens 25 g/g, besonders bevorzugt mindestens 26 g/g, ganz besonders bevorzugt mindestens 27 g/g, und üblicherweise nicht über 45 g/g.

Die Flüssigkeitsweiterleitung (SFC) der Polymerpartikel A beträgt üblicherweise mindestens $80x10^{-7}cm^3s/g$, vorzugsweise mindestens $90x10^{-7}cm^3s/g$, bevorzugt mindestens $100x10^{-7}cm^3s/g$, besonders bevorzugt mindestens $120x10^{-7}cm^3s/g$, ganz besonders bevorzugt mindestens $150x10^{-7}CM^3s/g$, und üblicherweise nicht über $500x10^{-7}cm^3s/g$.

Polymerpartikel B:

Vorzugsweise weisen weniger als 2 Gew.-%, besonders bevorzugt weniger als 1,5 Gew.-%, ganz besonders bevorzugt weniger als 1 Gew.-%, der Polymerpartikel B eine Partikelgröße von über 850 μm auf.

Vorzugsweise weisen mindestens 90 Gew.-%, bevorzugt mindestens 95 Gew.-%, besonders bevorzugt mindestens 98 Gew.-%, ganz besonders bevorzugt mindestens 99 Gew.-%, der Polymerpartikel B eine Partikelgröße von 150 bis 850 μm auf.

Die Polymerpartikel B weisen üblicherweise zu mindestens 16 Gew.-%, vorzugsweise zu mindestens 17 Gew.-%, bevorzugt zu mindestens 18 Gew.-%, besonders bevorzugt zu mindestens 19 Gew.-%, ganz besonders bevorzugt zu mindestens 20 Gew.-%, eine Partikelgröße von weniger als 300 μm auf.

Die Zentrifugenretentionskapazität (CRC) der Polymerpartikel B beträgt üblicherweise mehr als 28 g/g, mindestens 29 g/g, vorzugsweise mindestens 30 g/g, bevorzugt mindestens 31 g/g, besonders bevorzugt mindestens 32 g/g, ganz besonders bevorzugt mindestens 33 g/g, und üblicherweise nicht über 50 g/g.

Die Absorption unter einem Druck von 4,83 kPa (AUL0.7psi) der Polymerpartikel B beträgt üblicherweise mindestens 23 g/g, vorzugsweise mindestens 24 g/g, bevorzugt mindestens 25 g/g, besonders bevorzugt mindestens 26 g/g, ganz besonders bevorzugt mindestens 27 g/g, und üblichervveise nicht über 45 g/g.

Die Flüssigkeitsweiterleitung (SFC) der Polymerpartikel B beträgt üblicherweise mindestens $45x10^{-7}cm^3s/g$, vorzugsweise mindestens $50x10^{-7}cm^3s/g$, bevorzugt mindestens $60x10^{-7}cm^3s/g$, besonders bevorzugt mindestens $70x10^{-7}cm^3s/g$, ganz besonders bevorzugt mindestens $80x10^{-7}cm^3s/g$, und üblicherweise nicht über $500x10^{-7}cm^3s/g$.

Vorzugsweise sind die Polymerpartikel B mit einem wasserunlöslichen Metallphosphat beschichtet.

Wasserunlöslich bedeutet eine Löslichkeit von weniger als 1 g, vorzugsweise von weniger als 0,1 g, besonders bevorzugt von weniger als 0,01 g, in 100 ml Wasser bei 25˚C.

Geeignete wasserunlösliche Metallphosphate sind beispielsweise Phosphate, die im technischen Sinn als "Phosphate" angesehen werden können, wie Phosphatoxide, Phosphathydroxide, Phosphatsilikate, Phosphatfluoride oder dergleichen.

Bevorzugte wasserunlösliche Metallphosphate sind Pyrophosphate, Hydrogenphosphate und Phosphate des Kalziums, Magnesiums, Strontiums, Bariums, Zinks, Eisens, Aluminiums, Titans, Zirkoniums, Hafniums, Zinns, Cers, Scandiums, Yttriums oder Lanthans, sowie Gemische davon.

Bevorzugte Phosphate sind Kalziumhydrogenphosphat, Kalziumphosphat, Apatit, Thomasmehl, Berlinit und Rhenaniaphosphat. Besonders bevorzugt sind Kalziumhydrogenphosphat, Kalziumphosphat und Apatit, wobei der Begriff Apatit Fluor-, Hydroxy-, Chlor-, Karbonat- oder Karbonat-Fluor-Apatit bedeutet. Selbstverständlich können Gemische verschiedener wasserunlöslicher Metallphosphate eingesetzt werden.

Üblicherweise beträgt der Anteil des wasserunlöslichen Metallphosphats 0,001 bis 10 Gew.-%, vorzugsweise 0,01 bis 5 Gew.-%, besonders bevorzugt 0,05 bis 2,5 Gew.-%, bezogen auf die wasserabsorbierenden Polymerpartikel B.

Die Zentrifugenretentionskapazität (CRC) der mit wasserunlöslichen Metallphosphaten beschichteten Polymerpartikel B beträgt üblicherweise mehr als 29 g/g, mindestens 30 g/g, vorzugsweise mindestens 31 g/g, bevorzugt mindestens 32 g/g, besonders bevorzugt mindestens 33 g/g, ganz besonders bevorzugt mindestens 34 g/g, und üblicherweise nicht über 50 g/g.

Die Absorption unter einem Druck von 4,83 kPa (AUL0.7psi) der mit wasserunlöslichen Metallphosphaten beschichteten Polymerpartikel B beträgt üblicherweise mindestens 23 g/g, vorzugsweise mindestens 24 g/g, bevorzugt mindestens 25 g/g, besonders bevorzugt mindestens 26 g/g, ganz besonders bevorzugt mindestens 27 g/g, und üblicherweise nicht über 45 g/g.

Die Flüssigkeitsweiterleitung (SFC) der mit wasserunlöslichen Metallphosphaten beschichteten Polymerpartikel B beträgt üblicherweise mindestens $45x10^{-7}cm^3s/g$, vorzugsweise mindestens $50x10^{-7}cm^3s/g$, bevorzugt mindestens $60x10^{-7}cm^3s/g$, besonders bevorzugt mindestens $70x10^{-7}cm^3s/g$, ganz besonders bevorzugt mindestens $80x10^{-7}cm^3s/g$, und üblicherweise nicht über $500x10^{-7}cm^3s/g$.

Polymerpartikel C:

Vorzugsweise weisen weniger als 2 Gew.-%, besonders bevorzugt weniger als 1,5 Gew.-%, ganz besonders bevorzugt weniger als 1 Gew.-%, der Polymerpartikel C eine Partikelgröße von über 850 μm auf.

Vorzugsweise weisen mindestens 90 Gew.-%, bevorzugt mindestens 95 Gew.-%, besonders bevorzugt mindestens 98 Gew.-%, ganz besonders bevorzugt mindestens 99 Gew.-%, der Polymerpartikel C eine Partikelgröße von 150 bis 850 μm auf.

Die Polymerpartikel C weisen üblicherweise zu weniger als 15 Gew.-%, vorzugsweise zu weniger als 14 Gew.-%, bevorzugt zu weniger als 13 Gew.-%, besonders bevorzugt zu weniger als 12 Gew.-%, ganz besonders bevorzugt zu weniger als 11 Gew.-%, eine Partikelgröße von weniger als 300 μm auf.

Die Zentrifugenretentionskapazität (CRC) der Polymerpartikel C beträgt üblicherweise mindestens 30 g/g, vorzugsweise mindestens 31 g/g, bevorzugt mindestens 32 g/g, besonders bevorzugt mindestens 33 g/g, ganz besonders bevorzugt mindestens 34 g/g, und üblicherweise nicht über 50 g/g.

Die Absorption unter einem Druck von 4,83 kPa (AUL0.7psi) der Polymerpartikel C beträgt üblicherweise mindestens 23 g/g, vorzugsweise mindestens 24 g/g, bevorzugt mindestens 25 g/g, besonders bevorzugt mindestens 26 g/g, ganz besonders bevorzugt mindestens 27 g/g, und üblicherweise nicht über 45 g/g.

Die Flüssigkeitsweiterleitung (SFC) der Polymerpartikel C beträgt üblicherweise mindestens $45x10^{-7}cm^3s/g$, vorzugsweise mindestens $50x10^{-7}cm^3s/g$, bevorzugt mindestens $60x10^{-7}cm^3s/g$, besonders bevorzugt mindestens $70x10^{-7}cm^3s/g$, ganz besonders bevorzugt mindestens $80x10^{-7}cm^3s/g$, und üblicherweise nicht über $500x10^{-7}cm^3s/g$.

Ein weiterer Gegenstand der vorliegenden Erfindung ist ein Verfahren zur Herstellung wasserabsorbierender Polymere durch Polymerisation einer Monomerlösung, enthaltend

i) mindestens ein ethylenisch ungesättigtes, säuregruppentragendes Monomer,

ii) mindestens einen Vernetzer,

iii) gegebenenfalls ein oder mehrere mit i) copolymerisierbare ethylenisch und/oder allylisch ungesättigte Monomeren und

iv) gegebenenfalls ein oder mehrere wasserlösliche Polymeren, auf die die Monomere i), ii) und ggf. iii) zumindest teilweise aufgepfropft werden können,
wobei das dabei erhaltene Grundpolymer getrocknet, klassiert, mit

v) mindestens einem Nachvernetzer,
nachbehandelt, getrocknet und thermisch nachvernetzt wird, **dadurch gekennzeichnet, dass** die thermische Nachvernetzung abgebrochen wird nachdem die wasserabsorbierenden Polymerpartikel eine Zentrifugenretentionskapazität (CRC) von mindestens 26 g/g und einen Transportwert (TW) von mindestens 15.000 $cm^3s$ aufweisen,
wobei der Transportwert (TW) das Produkt aus Flüssigkeitsweiterleitung (SFC) und Dochtabsorption nach 60 Minuten ($DA_{60}$) multipliziert mit $10^7$ ist, und
wobei die Dochtabsorption nach 60 Minuten ($DA_{60}$) die Gewichtsmenge an 0,9 gew.-%iger Kochsalzlösung ist, die 70 g der wasserabsorbierenden Polymerpartikel in 60 Minuten aufnehmen, wobei sich die wasserabsorbierenden Polymerpartikel während der Messung in einem kreisrunden Gefäß mit einem Innendurchmesser von 6 cm befinden, welches unten mit einem Siebboden der Maschenweite 36 μm verschlossen ist, und der Siebboden drucklos mit 0,9 gew.-%iger Kochsalzlösung in Kontakt steht.

Geeignete Monomere i) sind beispielsweise ethylenisch ungesättigte Carbonsäuren, wie Acrylsäure, Methacrylsäure, Maleinsäure, Fumarsäure und Itaconsäure, oder deren Derivate, wie Acrylamid, Methacrylamid, Acrylsäureester und Methacrylsäureester. Besonders bevorzugte Monomere sind Acrylsäure und Methacrylsäure. Ganz besonders bevorzugt ist Acrylsäure.

Die wasserabsorbierenden Polymere sind vernetzt, d.h. die Polymerisation wird in Gegenwart von Verbindungen mit mindestens zwei polymerisierbaren Gruppen, die in das Polymernetzwerk radikalisch einpolymerisiert werden können, durchgeführt. Geeignete Vernetzer ii) sind beispielsweise Ethylenglykoldimethacrylat, Diethylenglykoldiacrylat, Allylmethacrylat, Trimethylolpropantriacrylat, Triallylamin, Tetraallyloxyethan, wie in EP-A 530 438 beschrieben, Di- und Triacrylate, wie in EP-A 547 847, EP-A 559 476, EP-A 632 068, WO 93/21237, WO 03/104299, WO 03/104300, WO

03/104301 und in der deutschen Patentanmeldung mit dem Aktenzeichen 103 31 450.4 beschrieben, gemischte Acrylate, die neben Acrylatgruppen weitere ethylenisch ungesättigte Gruppen enthalten, wie in den deutschen Patentanmeldungen mit den Aktenzeichen 103 31 456.3 und 103 55 401.7 beschrieben, oder Vernetzermischungen, wie beispielsweise in DE-A 195 43 368, DE-A 196 46 484, WO 90/15830 und WO 02/32962 beschrieben.

Geeignete Vernetzer ii) sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A 343 427 beschrieben sind. Weiterhin geeignete Vernetzer ii) sind Pentaerythritoldi- Pentaerythritoltri- und Pentaerythritoltetraallylether, Polyethylenglykoldiallylether, Ethylenglykoldiallylether, Glyzerindi- und triallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1000 aufweist.

Besonders vorteilhafte Vernetzer ii) sind jedoch Di- und Triacrylate des 3- bis 15-fach ethoxylierten Glyzerins, des 3- bis 15-fach ethoxylierten Trimethylolpropans, inbesondere Di- und Triacrylate des 3-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des 40-fach ethoxylierten Glyzerins oder Trimethylolpropans.

Ganz besonders bevorzugte Vernetzer ii) sind die mit Acrylsäure oder Methacrylsäure zu Di- oder Triacrylaten veresterten mehrfach ethoxylierten und/oder propoxylierten Glyzerine wie sie beispielsweise in der älteren deutschen Anmeldung mit Aktenzeichen DE 103 19 462.2 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5- fach ethoxylierten und/oder propoxylierten Glyzerins. Am meisten bevorzugt sind die Triacrylate des 3- bis 5-fach ethoxylierten und/oder propoxylierten Glyzerins. Diese zeichnen sich durch besonders niedrige Restgehalte (typischerweise unter 10 ppm) im wasserabsorbierenden Polymer aus und die wässrigen Extrakte der damit hergestellten wasserabsorbierenden Polymere weisen eine fast unveränderte Oberflächenspannung (typischerweise mindestens 0,068 N/m) im Vergleich zu Wasser gleicher Temperatur auf.

Mit den Monomeren i) copolymerisierbare ethylenisch ungesättigte Monomere iii) sind beispielsweise Acrylamid, Methacrylamid, Crotonsäureamid, Dimethylaminoethylmethacrylat, Dimethylaminoethylacrylat, Dimethylaminopropylacrylat, Diethylaminopropylacrylat, Dimethylaminobutylacrylat, Dimethylaminoethylmethacrylat, Diethylaminoethylmethacrylat, Dimethylaminoneopentylacrylat und Dimethylaminoneopentylmethacrylat.

Als wasserlösliche Polymere iv) können Polyvinylalkohol, Polyvinylpyrrolidon, Stärke, Stärkederivate, Polyglykole oder Polyacrylsäuren, vorzugsweise Polyvinylalkohol und Stärke, eingesetzt werden.

Die Herstellung eines geeigneten Grundpolymers sowie weitere geeignete hydrophile ethylenisch ungesättigte Monomere i) werden in DE-A 199 41 423, EP-A 686 650, WO 01/45758 und WO 03/104300 beschrieben.

Die Umsetzung wird vorzugsweise in einem Kneter, wie beispielsweise in WO 01/38402 beschrieben, oder auf einem Bandreaktor, wie beispielsweise in EP-A 955 086 beschrieben, durchgeführt.

Die Säuregruppen der erhaltenen Hydrogele sind vorzugsweise zu größer 60 mol-%, bevorzugt zu größer 61 mol-%, besonders bevorzugt zu größer 62 mol-%, ganz besonders bevorzugt zu größer 63 mol%, und vorzugsweise zu höchstens 70 mol-%, bevorzugt zu höchstens 69 mol-%, besonders bevorzugt zu höchstens 68 mol-%, ganz besonders bevorzugt zu höchstens 67 mol-%, neutralisiert, wobei die üblichen Neutralisationsmittel verwendet werden können, beispielsweise Ammoniak, Amine, wie Ethanolamin, Diethanolamin, Triethanolamin oder Dimethylaminoethanolamin, vorzugsweise Alkalimetallhydroxide, Alkalimetalloxide, Alkalimetallcarbonate oder Alkalimetallhydrogencarbonate sowie deren Mischungen, wobei Natrium und Kalium als Alkalimetalle besonders bevorzugt sind, ganz besonders bevorzugt jedoch Natrium-hydroxid, Natriumcarbonat oder Natriumhydrogencarbonat sowie deren Mischungen. Üblicherweise wird die Neutralisation durch Einmischung des Neutralisationsmittels als wässrige Lösung oder bevorzugt auch als Feststoff erreicht.

Die Neutralisation kann nach der Polymerisation auf der Stufe des Hydrogels durchgeführt werden. Es ist aber auch möglich bis zu 40 mol-%, vorzugsweise 10 bis 30 mol-%, besonders bevorzugt 15 bis 25 mol-%, der Säureguppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Hydrogels einzustellt wird. Die Monomerlösung kann durch Einmischen des Neutralisationsmittels neutralisiert werden, entweder auf einen vorbestimmten Vorneutralisationgrad mit anschliessender Nachneutralisation auf den Endwert nach oder während der Polymerisationsreaktion, oder die Monomerlösung wird durch Einmischen des Neutralisationsmittels vor der Polymerisation direkt auf den Endwert eingestellt. Das Hydrogel kann mechanisch zerkleinert werden, beispielsweise mittels eines Fleischwolfes, wobei das Neutralisationsmittel aufgesprüht, übergestreut oder aufgegossen und dann sorgfältig untergemischt werden kann. Dazu kann die erhaltene Gelmasse noch mehrmals zur Homogenisierung gewolft werden.

Bei zu geringem Neutralisationsgrad kommt es bei der anschliessenden Trocknung sowie während der anschließenden Nachvernetzung des Grundpolymeren zu unerwünschten thermischen Vernetzungseffekten, welche die Zentrifugenretentionskapazität (CRC) des wasserabsorbierenden Polymeren stark verringern können, bis hin zur Unbrauchbarkeit.

Bei zu hohem Neutralisationsgrad kommt es jedoch zu einer weniger effizienten Nachvernetzung, was zu einer verringerten Flüssigkeitsweiterleitung (SFC) des gequollenen Hydrogels führt.

Ein optimales Ergebnis erhält man hingegen wenn man den Neutralisationsgrad des Grundpolymeren so einstellt, dass man eine effiziente Nachvernetzung erreicht und somit eine hohe Flüssigkeitsweiterleitung (SFC), wobei man gleichzeitig aber noch so weit neutralisiert, dass man das Hydrogel bei der Herstellung in einem gewöhnlichen Bandtrockner oder anderen im technischen Maßstab üblichen Trocknungsapparaturen ohne Verlust an Zentrifugenretentionskapazität (CRC) trocknen kann.

Das neutralisierte Hydrogel wird dann mit einem Band-, Wirbelschicht-, Schacht- oder Walzentrockner getrocknet bis der Restfeuchtegehalt vorzugsweise unter 10 Gew.-%, insbesondere unter 5 Gew.-% liegt, wobei der Wassergehalt gemäß der von der EDANA (European Disposables and Nonwovens Association) empfohlenen Testmethode Nr. 430.2-02 "Moisture content" bestimmt wird. Das getrocknete Hydrogel wird hiernach gemahlen und gesiebt, wobei zur Mahlung üblicherweise Walzenstühle, Stiftmühlen oder Schwingmühlen eingesetzt werden können, wobei Siebe mit zur Herstellung der wasserabsorbierenden Polymerpartikel A, B und C notwendigen Maschenweiten verwendet werden.

Die Grundpolymere werden anschließend nachvernetzt. Hierzu geeignete Nachvernetzer v) sind Verbindungen, die mindestens zwei Gruppen enthalten, die mit den Carboxylatgruppen der Polymere kovalente Bindungen bilden können. Geeignete Verbindungen sind beispielsweise Alkoxysiliylverbindungen, Polyaziridine, Polyamine, Polyamidoamine, Di- oder Polyglycidylverbindungen, wie in EP-A 083 022, EP-A 543 303 und EP-A 937 736 beschrieben, mehrwertige Alkohole, wie in DE-C 33 14 019, DE-C 35 23 617 und EP-A 450 922 beschrieben, oder β-Hydroxyalkylamide, wie in DE-A 102 04 938 und US 6,239,230 beschrieben. Geeignet sind ferner Verbindungen mit gemischter Funktionalität, wie Glycidol, 3-Ethyl-3-Oxetanmethanol (Trimethylolpropanoxetan), wie in EP-A 1 199 327 beschrieben, Aminoethanol, Diethanolamin, Triethanolamin oder Verbindungen, die nach der ersten Reaktion eine weitere Funktionalität ausbilden, wie Ethylenoxid, Propylenoxid, Isobutylenoxid, Aziridin, Azetidin oder Oxetan.

Desweiteren sind in DE-A 40 20 780 zyclische Karbonate, in DE-A 198 07 502 2-Oxazolidon und dessen Derivate, wie N-(2-Hydroxyethyl)-2-oxazolidon, in DE-A 198 07 992 Bis- und Poly-2-oxazolidone, in DE-A 198 54 573 2-Oxotetrahydro-1,3-oxazin und dessen Derivate, in DE-A 198 54 574 N-Acyl-2-Oxazolidone, in DE-A 102 04 937 zyklische Harnstoffe, in der deutschen Patentanmeldung mit dem Aktenzeichen 103 34 584.1 bizyklische Amidacetale, in EP-A 1 199 327 Oxetane und zyklische Harnstoffe und in WO 03/031482 Morpholin-2,3-dion und dessen Derivate als geeignete Nachvernetzer v) beschrieben.

Die Nachvernetzung wird üblicherweise so durchgeführt, dass eine Lösung des Nachvernetzers auf das Hydrogel oder die trockenen Grundpolymerpartikel aufgesprüht wird. Im Anschluss an das Aufsprühen wird thermisch getrocknet, wobei die Nachvernetzungsreaktion sowohl vor als auch während der Trocknung stattfinden kann.

Bevorzugte Nachvernetzer v) sind Amidacetale oder Carbaminsäureester der allgemeinen Formel I

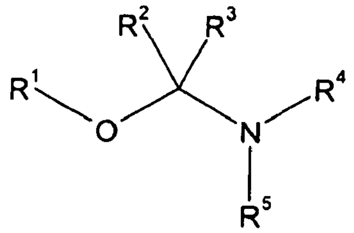

(I)

worin

$R^1$ $C_1$-$C_{12}$-Alkyl, $C_2$-$C_{12}$-Hydroxyalkyl, $C_2$-$C_{12}$-Alkenyl oder $C_6$-$C_{12}$-Aryl,

$R^2$ X oder $OR^6$

$R^3$ Wasserstoff, $C_1$-$C_{12}$-Alkyl, $C_2$-$C_{12}$-Hydroxyalkyl, $C_2$-$C_{12}$-Alkenyl oder $C_6$-$C_{12}$-Aryl, oder X,

$R^4$ $C_1$-$C_{12}$-Alkyl, $C_2$-$C_{12}$-Hydroxyalkyl, $C_2$-$C_{12}$-Alkenyl oder $C_6$-$C_{12}$-Aryl,

$R^5$ Wasserstoff, $C_1$-$C_{12}$-Alkyl, $C_2$-$C_{12}$-Hydroxyalkyl, $C_2$-$C_{12}$-Alkenyl, $C_1$-$C_{12}$-Acyl oder $C_6$-$C_{12}$-Aryl.

$R^6$ $C_1$-$C_{12}$-Alkyl, $C_2$-$C_{12}$-Hydroxyalkyl, $C_2$-$C_{12}$-Alkenyl oder $C_6$-$C_{12}$-Aryl und

X ein für die Reste $R^2$ und $R^3$ gemeinsamer Carbonyl-Sauerstoff

bedeuten, wobei $R^1$ und $R^4$ und/oder $R^5$ und $R^6$ ein verbrücktes $C_2$-$C_6$-Alkandiyl sein können, und wobei die obengenannte Reste $R^1$ bis $R^6$ noch insgesamt über ein bis zwei freie Valenzen verfügen können und mit diesen freien Valenzen mit mindestens einem geeigneten Grundkörper verbunden sein können,
oder mehrwertige Alkohole, wobei der mehrwertige Alkohol vorzugsweise ein Molekulargewicht von weniger als 100 g/mol, bevorzugt von weniger als 90 g/mol, besonders bevorzugt von weniger als 80 g/mol, ganz besonders bevorzugt von weniger als 70 g/mol, pro Hydroxylgruppe sowie keine vincinalen, geminalen, sekundären oder tertiären Hydroxylgruppen aufweist, und mehrwertige Alkohole entweder Diole der allgemeinen Formel IIa

$$HO\text{-}R^6\text{-}OH \qquad (IIa)$$

worin $R^6$ entweder einen unverzweigten Dialkylrest der Formel $-(CH_2)_n-$, wobei n eine ganze Zahl von 3 bis 20, bevorzugt 3 bis 12 ist, wobei 4 weniger bevorzugt ist, bedeutet und beide Hydroxylgruppen endständig sind, oder $R^6$ einen unverzweigten, verzweigten oder zyklischen Dialkylrest bedeutet, oder Polyole der allgemeinen Formel IIb

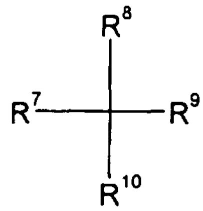

(IIb)

worin die Reste $R^7$, $R^8$, $R^9$, $R^{10}$ unabhängig voneinander Wasserstoff, Hydroxyl, Hydroxymethyl, Hydroxyethyloxymethyl, 1-Hydroxyprop-2-yloxymethyl, 2-Hydroxypropyloxymethyl, Methyl, Ethyl, n-Propyl, Isopropyl, n-Butyl, n-Pentyl, n-Hexyl, 1,2-Dihydroxyethyl, 2-Hydroxyethyl, 3-Hydroxypropyl oder 4-Hydroxybutyl bedeuten und insgesamt 2, 3, oder 4, bevorzugt 2 oder 3, Hydroxylgruppen vorhanden sind, und nicht mehr als einer der Reste $R^7$, $R^8$, $R^9$, oder $R^{10}$ gleich Hydroxyl bedeutet, sind,
oder zyklische Karbonate der allgemeinen Formel III

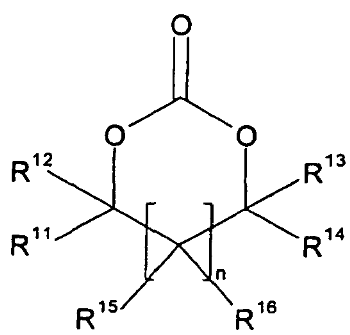

(III)

worin $R^{11}$, $R^{12}$, $R^{13}$, $R^{14}$, $R^{15}$ und $R^{16}$ unabhängig voneinander Wasserstoff, Methyl, Ethyl, n-Propyl, Isopropyl, n-Butyl, sek.-Butyl oder Isobutyl, und n entweder 0 oder 1 ist,
oder Bisoxazoline der allgemeinen Formel IV

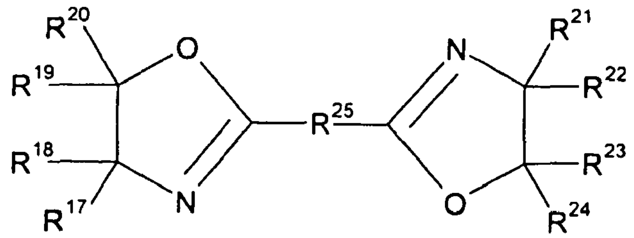

(IV)

worin $R^{17}$, $R^{18}$, $R^{19}$, $R^{20}$, $R^{21}$, $R^{22}$, $R^{23}$ und $R^{24}$ unabhängig voneinander Wasserstoff, Methyl, Ethyl, n-Propyl, Isopropyl, n-Butyl, sek.-Butyl oder Isobutyl, und $R^{25}$ eine Einfachbindung, einen linearen, verzweigten oder zyklischen $C_1$-$C_{12}$-Dialkylrest, oder einen Polyalkoxydiylrest darstellt, welcher aus ein bis zehn Ethylenoxid- und/oder Propylenoxideinheiten aufgebaut ist, wie sie beispielsweise Polyglykoldicarbonsäuren aufweisen.

Die bevorzugten Nachvernetzer v) sind außerordentlich selektiv. Neben- und Folgereaktionen, die zu flüchtigen und damit übelriechenden Verbindungen führen sind minimiert. Die mit den bevorzugten Nachvernetzern v) hergestellten wasserabsorbierenden Polymere sind daher auch im angefeuchteten Zustand geruchsneutral.

Dagegen können Epoxyverbindungen bei hohen Temperaturen und Anwesenheit geeigneter Katalysatoren verschiedene Umlagerungsreaktionen eingehen, die beispielsweise zu Aldehyden oder Ketonen führen. Diese können dann weitere Folgereaktionen eingehen, wodurch sich letztendlich übelriechende Verunreinigungen bilden, die wegen ihres Geruchs in Hygieneartikeln unerwünscht sind. Daher sind Epoxyverbindungen oberhalb einer Temperatur von ca. 140 bis 150°C weniger geeignet für die Nachvernetzung. Bei Amino- bzw. Iminogruppen enthaltenden Nachvernetzern v) kommt es bei ähnlichen Temperaturen zu noch unübersichtlicheren Umlagerungsreaktionen, wodurch leicht übelriechende Spurenverunreinigungen und bräunliche Produktverfärbungen auftreten.

Mehrwertige Alkohole als Nachvernetzer v) benötigen aufgrund ihrer geringen Reaktivität hohe Nachvernetzungstemperaturen. Alkohole, die vincinale, geminale, sekundäre und tertiäre Hydroxylgruppen aufweisen, bilden dabei im Hygienebereich unerwünschte Nebenprodukte, die zu unangenehmen Gerüchen und/oder Verfärbungen des betreffenden Hygieneartikels während der Herstellung oder des Gebrauchs führen.

Bevorzugte Nachvernetzer v) der allgemeinen Formel I sind 2-Oxazolidone, wie 2-Oxazolidon und N-Hydroxyethyl-2-oxazolidon, N-Methyl-2-Oxazolidon, N-Acyl-2-oxazolidone, wie N-Acetyl-2-oxazolidon, 2-Oxotetrahydro-1,3-oxazin, bicyclische Amidacetale, wie 5-Methyl-1-aza-4,6-dioxa-bicyclo[3.3.0]octan, 1-Aza-4,6-dioxa-bicyclo[3.3.0]octan und 5-Isopropyl-1-aza-4,6-dioxa-bicyclo [3.3.0] octan, Bis-2-oxazolidone und Poly-2-oxazolidone.

Besonders bevorzugte Nachvernetzer v) der allgemeinen Formel I sind 2-Oxazolidon, N-Methyl-2-Oxazolidon, N-Hydroxyethyl-2-oxazolidon und N-Hydroxypropyl-2-oxazolidon.

Bevorzugte Nachvernetzer v) der allgemeinen Formel IIa sind 1,3-Propandiol, 1,5-Pentandiol, 1,6-Hexandiol und 1,7-Heptandiol. Weitere Beispiele für Nachvernetzer der Formel IIa sind 1,3-Butandiol, 1,8-Octandiol, 1,9-Nonandiol und 1,10-Decandiol.

Die Diole IIa sind vorzugsweise wasserlöslich, wobei sich die Diole der allgemeinen Formel IIa bei 23°C zu mindestens 30 Gew.-%, bevorzugt zu mindestens 40 Gew.-%, besonders bevorzugt zu mindestens 50 Gew.-%, ganz besonders bevorzugt mindestens zu 60 Gew.-%, in Wasser lösen, wie beispielsweise 1,3-Propandiol und 1,7-Heptandiol. Noch mehr bevorzugt sind solche Nachvernetzer die bei 25°C flüssig sind.

Bevorzugte Nachvernetzer v) der allgemeinen Formel IIb sind Butan-1,2,3-triol, Butan-1,2,4-triol, Glyzerin, Trimethylolpropan, Trimethylolethan, Pentaerythrit, pro Molekül 1-bis 3-fach-ethoxyliertes Glyzerin, Trimethylolethan oder Trimethylolpropan und pro Molekül 1- bis 3-fach-propoxyliertes Glyzerin, Trimethylolethan oder Trimethylolpropan. Weiterhin bevorzugt sind 2-fach ethoxyliertes oder propoxyliertes Neopentylglykol. Besonders bevorzugt sind 2-fach und 3-fach ethoxyliertes Glyzerin und Trimethylolpropan.

Bevorzugte mehrwertige Alkohole IIa und IIb weisen bei 23°C eine Viskosität von weniger als 3000 mPas, vorzugsweise weniger als 1500 mPas, bevorzugt weniger als 1000 mPas, besonders bevorzugt weniger als 500 mPas, ganz besonders bevorzugt weniger als 300 mPas, auf.

Besonders bevorzugte Nachvernetzer v) der allgemeinen Formel III sind Ethylencarbonat und Propylencarbonat.

Ein besonders bevorzugter Nachvernetzer v) der allgemeinen Formel IV ist 2,2'-Bis(2-oxazolin).

Der mindestens eine Nachvernetzer v) wird typischerweise in einer Menge von höchstens 0,30 Gew.-%, vorzugsweise von höchstens 0,15 Gew.-%, besonders bevorzugt von 0,001 bis 0,095 Gew.-%, jeweils bezogen auf das Grundpolymer, als wässrige Lösung eingesetzt.

Es kann ein einzelner Nachvernetzer v) aus der obigen Auswahl verwendet werden oder beliebige Gemische verschiedener Nachvernetzer.

Die wässrige Nachvernetzerlösung kann neben dem mindestens einen Nachvernetzer v) typischerweise noch ein Cosolvens enthalten.

Technisch gut geeignete Cosolventien sind $C_1$-$C_6$-Alkohole, wie Methanol, Ethanol, n-Propanol, Isopropanol, n-Butanol, sec-Butanol, tert-Butanol oder 2-Methyl-1-Propanol, $C_2$-$C_5$-Diole, wie Ethylenglykol, 1,2-Propylenglykol oder 1,4-Butandiol, Ketone, wie Aceton, oder Carbonsäureester, wie Essigsäureethylester. Nachteilig an vielen dieser Cosolventien ist, dass sie typische Eigengerüche aufweisen.

Das Cosolvens selbst ist unter den Reaktionsbedingungen idealerweise kein Nachvernetzer. Es kann jedoch im Grenzfall und abhängig von Verweilzeit und Temperatur dazu kommen, dass das Cosolvens teilweise zur Vernetzung beiträgt. Dies ist insbesondere dann der Fall, wenn der Nachvernetzer v) relativ träge ist und daher auch selbst sein Cosolvens bilden kann, wie beispielsweise bei Einsatz zyklischer Karbonate der allgemeinen Formel III, Diole der allgemeinen Formel IIa oder Polyole der allgemeinen Formel IIb. Solche Nachvernetzer v) können im Gemisch mit reaktiveren Nachvernetzern v) auch in der Funktion als Cosolvens eingesetzt werden, da die eigentliche Nachvernetzungsreaktion dann bei niedrigeren Temperaturen und/oder kürzeren Verweilzeiten als in Abwesenheit des reaktiveren Vernetzers v) durchgeführt werden kann. Da das Cosolvens in relativ großen Mengen verwendet wird und auch teilweise im Produkt verbleibt, darf es nicht toxisch sein.

Im erfindungsgemäßen Verfahren eignen sich die Diole der allgemeinen Formel IIa, die Polyole der allgemeinen Formel IIb, sowie die zyklischen Karbonate der allgemeinen Formel III auch als Cosolventien. Diese Funktion erfüllen sie in Gegenwart eines reaktiven Nachvernetzers v) der allgemeinen Formel I und/oder IV und/oder eines Di- oder Triyglycidylvernetzers. Bevorzugte Cosolventien im erfindungsgemäßen Verfahren sind jedoch insbesondere die Diole der allgemeinen Formel IIa, insbesondere, wenn die Hydroxygruppen sterisch durch Nachbargruppen an einer Reaktion behindert werden. Solche Diole eignen sich zwar prinzipiell auch als Nachvernetzer v), erfordern dazu jedoch deutlich höhere Reaktionstemperaturen oder gegebenenfalls höhere Einsatzmengen als sterisch ungehinderte Diole. Geeignete sterisch gehinderte und damit reaktionsträge Diole sind auch Diole mit tertiären Hydroxylgruppen.

Beispiele für solche sterisch gehinderten und daher als Cosolvens besonders bevorzugten Diole der allgemeinen Formel IIa sind 2,2-Dimethyl-1,3-propandiol (Neopentylglykol), 2-Ethyl-1,3-Hexandiol, 2-Methyl-1,3-Propandiol und 2,4-Dimethylpentan-2,4-diol.

Weitere im erfindungsgemäßen Verfahren besonders bevorzugte Cosolventien sind die Polyole der allgemeinen Formel IIb. Insbesondere bevorzugt sind hiervon die 2- bis 3-fach alkoxylierten Polyole. Besonders geeignet als Cosolventien sind aber auch 3- bis 15-fach, ganz besonders 5- bis 10-fach ethoxylierte Polyole auf der Basis von Glyzerin, Trimethylolpropan, Trimethylolethan oder Pentaerythrit. Besonders gut geeignet ist 7-fach ethoxyliertes Trimethylolpropan.

Weitere geeignete Cosolventien sind Di(trimethylolpropan) sowie 5-Ethyl-1,3-dioxan-5-methanol.

Besonders bevorzugte Kombinationen aus wenig reaktivem Nachvernetzer v) als Cosolvens und reaktivem Nachvernetzter v) sind Kombinationen von bevorzugten mehrwertigen Alkoholen, Diolen der allgemeinen Formel IIa und Polyolen der allgemeinen Formel IIb, mit Amidacetalen oder Carbaminsäureestern der allgemeinen Formel I.

Ganz besonders bevorzugte Kombinationen sind 2-Oxazolidon/1,3-Propandiol und N-(2-Hydroxyethyl)-2-oxazolidon/1,3-Propandiol.

Weitere ganz besonders bevorzugte Kombinationen sind 2-Oxazolidon oder N-(2-Hydroxyethyl)-2-oxazolidon als Reaktivvernetzer kombiniert mit 1,5-Pentandiol oder 1,6-Hexandiol oder 2-Methyl-1,3-Propandiol oder 2,2-Dimethyl-1,3-Propandiol, gelöst in Wasser und/oder Isopropanol als nicht-reaktivem Lösemittel.

Der mindestens eine Nachvernetzer v) hat vorzugsweise einen Siedepunkt von höchstens 160°C, besonders bevorzugt von höchstens 140°C, ganz besonders bevorzugt von höchstens 120°C, oder vorzugsweise einen Siedepunkt von mindestens 200°C, besonders bevorzugt von mindestens 220°C, ganz besonders bevorzugt von mindestens 250°C.

Das Cosolvens hat vorzugsweise einen Siedepunkt von höchstens 160°C, besonders bevorzugt von höchstens 140°C, ganz besonders bevorzugt von höchstens 120°C, oder vorzugsweise einen Siedepunkt von mindestens 200°C, besonders bevorzugt von mindestens 220°C, ganz besonders bevorzugt von mindestens 250°C.

Im erfindungsgemäßen Verfahren besonders gut geeignete Cosolventien sind daher auch solche, die ein niedrig siedendes Azeotrop mit Wasser oder einem zweiten Cosolvens bilden. Bevorzugt liegt der Siedepunkt dieses Azeotrops bei höchstens 160°C, besonders bevorzugt höchstens 140°C, ganz besonders bevorzugt bei höchstens 120°C. Weiterhin gut geeignet sind wasserdampfflüchtige Cosolventien, da diese ganz oder teilweise mit dem bei der Trocknung verdampfenden Wasser entfernt werden.

Nachvernetzer v) und Cosolventien mit einem mittleren Siedepunkt führen überraschenderweise zu wasserabsorbierenden Polymerpartikeln mit einem unerwünschten chemischen Geruch.

Häufig beträgt die Konzentration des Cosolvens in der wässrigen Nachvernetzerlösung, von 15 bis 50 Gew.-%, vorzugsweise von 15 bis 40 Gew.-%, besonders bevorzugt von 20 bis 35 Gew.-% , bezogen auf die Nachvernetzerlösung. Bei Cosolventien, die mit Wasser nur begrenzt mischbar sind, wird man vorteilhaft die wässrige Nachvernetzerlösung so einstellen, dass nur eine Phase vorliegt, gegebenenfalls durch Erniedrigung der Konzentration des Cosolvens.

In einer bevorzugten Ausführungsform wird kein Cosolvens eingesetzt. Der mindestens eine Nachvernetzer v) wird dann nur als Lösung in Wasser angewandt, gegebenenfalls unter Zusatz eines Deagglomerationshilfsmittels.

Die Konzentration des mindestens einen Nachvernetzers v) in der wässrigen Nachvernetzerlösung, beträgt beispielsweise 1 bis 20 Gew.-%, vorzugsweise 1,5 bis 10 Gew.-%, besonders bevorzugt 2 bis 5 Gew.-%, bezogen auf die Nachvernetzerlösung.

Die Gesamtmenge der Nachvernetzerlösung bezogen auf Grundpolymer beträgt üblicherweise von 0,3 bis 15 Gew.-%, vorzugsweise von 2 bis 6 Gew.-%.

In einer bevorzugten Ausführungsform wird dem Grundpolymer ein Tensid als Deagglomerationshilfsmittel, beispielsweise Sorbitanmonoester, wie Sorbitanmonococoat und Sorbitanmonolaurat, oder ethoxylierte Varianten davon zugesetzt. Weitere sehr geeignete Deagglomerationshilfsmittel stellen die ethoxylierten und alkoylierten Derivate des 2-Propylheptanols dar, die unter den Handelsmarken Lutensol XL® und Lutensol XP® vertrieben werden (BASF AG, DE). Das Deagglomerationshilfsmittel kann getrennt dosiert oder der Nachvernetzerlösung zugesetzt werden. Vorzugsweise wird das Deagglomerationshilfsmittel der Nachvernetzerlösung zugesetzt.

Die Einsatzmenge des Deagglomerationshilfsmittels bezogen auf Grundpolymer beträgt beispielsweise 0 bis 0,01 Gew.-%, vorzugsweise 0 bis 0,005 Gew.-%, besonders bevorzugt 0 bis 0,002 Gew-%. Vorzugsweise wird das Deagglomerationshilfsmittel so dosiert, dass die Oberflächenspannung eines wässrigen Extrakts des gequollenen Grundpolymers und/oder des gequollenen nachvernetzten wasserabsorbierenden Polymeren bei 23°C mindestens 0,060 N/m, vorzugsweise mindestens 0,062 N/m, besonders bevorzugt mindestens 0,065 N/m, und vorteilhaft höchstens 0,072 N/m beträgt.

Das im erfindungsgemäßen Verfahren eingesetzte getrocknete Grundpolymer weist typischerweise einen Restfeuchtegehalt nach der Trocknung und vor Aufbringen der Nachvernetzungslösung von 0 bis 13 Gew.%, bevorzugt 2 bis 9 Gew.% auf. Optional kann dieser Feuchtegehalt aber auch beispielsweise durch Aufbringen von Wasser in einem vorgeschalteten Sprühmischer auf bis zu 75 Gew.% erhöht werden. Der Feuchtegehalt wird gemäß der von der EDANA (European Disposables and Nonwovens Association) empfohlenen Testmethode Nr. 430.2-02 "Moisture content" bestimmt. Eine solche Erhöhung des Feuchtegehalts führt zu einer leichten Vorquellung des Grundpolymers und verbessert die Verteilung des Vernetzers auf der Oberfläche sowie die Durchdringung der Partikel.

Die im erfindungsgemäßen Verfahren einsetzbaren Sprühdüsen unterliegen keiner Beschränkung. Derartigen Düsen kann die zu versprühende Flüssigkeit unter Druck zugeführt werden. Die Zerteilung der zu versprühenden Flüssigkeit kann dabei dadurch erfolgen, dass sie nach Erreichen einer bestimmten Mindestgeschwindigkeit in der Düsenbohrung entspannt wird. Ferner können für den erfindungsgemäßen Zweck auch Einstoffdüsen, wie beispielsweise Schlitzdüsen oder Drallkammern (Vollkegeldüsen) verwendet werden (beispielsweise von Düsen-Schlick GmbH, DE, oder von Spraying Systems Deutschland GmbH, DE). Derartige Düsen sind auch in der EP-A 534 228 und der EP-A 1 191 051 beschrieben.

Im Anschluß an das Aufsprühen wird das Polymerpulver thermisch getrocknet, wobei die Nachvernetzungsreaktion sowohl vor, während oder nach der Trocknung stattfinden kann.

Das Aufsprühen der Nachvernetzerlösung wird vorzugsweise in Mischern mit bewegten Mischwerkzeugen, wie Schneckenmischer, Paddelmischer, Scheibenmischer, Pflugscharmischer und Schaufelmischer, durchgeführt. Besonders bevorzugt sind Vertikalmischer, ganz besonders bevorzugt sind Pflugscharmischer und Schaufelmischer. Geeignete Mischer sind beispielsweise Lödige®-Mischer, Bepex®-Mischer, Nauta®-Mischer, Processall®-Mischer und Schugi®-Mischer.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex®-Trockner und Nara®-Trockner. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Es kann aber auch beispielsweise eine azeotrope Destillation als Trocknungsverfahren benutzt werden.

Besonders bevorzugt wird die Nachvernetzerlösung in einem Hochgeschwindigkeitsmischer, beispielsweise vom Typ Schugi-Flexomix® oder Turbolizer®, auf das Grundpolymer aufgebracht und in einem Reaktionstrockner, beispielsweise vom Typ Nara-Paddle-Dryer® oder einem Scheibentrockner, thermisch nachvernetzt. Das eingesetzte Grundpolymer kann aus vorhergehenden Prozessschritten noch eine Temperatur von 10 bis 120°C aufweisen, die Nachvernetzerlösung kann eine Temperatur von 0 bis 150 °C aufweisen. Insbesondere kann Nachvernetzerlösung zur Verminderung der Viskosität erwärmt werden. Bevorzugt zur Nachvernetzung und Trocknung ist dabei der Temperaturbereich von 30 bis 220°C, insbesondere 150 bis 210 °C, besonders bevorzugt 160 bis 190°C. Die bevorzugte Verweilzeit bei dieser Temperatur im Reaktionsmischer oder Trockner beträgt unter 100 Minuten, besonders bevorzugt unter 70 Minuten, am meisten bevorzugt unter 40 Minuten.

Der Nachvernetzungstrockner wird während der Trocknungs- und Nachvernetzungsreaktion mit Luft gespült, um die Dämpfe zu entfernen. Zur Unterstützung der Trocknung werden der Trockner und die angeschlossenen Aggregate

möglichst vollständig beheizt.

Selbstverständlich können mit den Dämpfen abgeführte Cosolventien außerhalb des Reaktionstrockners wieder kondensiert und gegebenenfalls rezyklisiert werden.

Nach Abschluss der Reaktionstrocknung werden die getrockneten wasserabsorbierenden Polymerpartikel abgekühlt. Vorzugsweise überführt man dazu das warme und trockene Polymer im kontinuierlichen Betrieb in einen nachgeschalteten Kühler. Dieser kann beispielsweise ein Scheibenkühler, ein Nara-Paddle-Kühler oder ein Schneckenkühler sein. Gekühlt wird dabei über die Wände und gegebenenfalls die Rührorgane des Kühlers, welche von einem geeigneten Kühlmedium wie beispielsweise Warm- oder Kaltwasser durchströmt werden. Zweckmäßig können im Kühler Wasser oder wässrige Lösungen von Additiven aufgesprüht werden; dadurch erhöht sich die Effizienz der Kühlung (partielle Wasserverdampfung) und der Restfeuchtegehalt im Fertigprodukt kann auf 0 bis 6 Gew.-%, vorzugsweise 0,01 bis 4 Gew.-%, besonders bevorzugt 0,1 bis 3 Gew.-%, eingestellt werden. Der erhöhte Restfeuchtegehalt verringert den Staubgehalt des Produkts.

Optional kann jedoch im Kühler auch nur gekühlt werden und die Zugabe von Wasser und Additiven in einem nachgeschalteten separaten Mischer durchgeführt werden. Die Kühlung stoppt die Reaktion durch Unterschreiten der Reaktionstemperatur und die Temperatur braucht insgesamt nur so weit abgesenkt werden, dass das Produkt sich problemlos in Plastiksäcke oder in Silo-LKW abpacken lässt.

Die wasserabsorbierenden Polymerpartikel können mit wasserunlöslichen Metallphosphaten beschichtet werden, wie in WO 02/060983 beschrieben.

Dazu können die wasserunlöslichen Metallphosphate als Pulver oder als Dispersion in einem geeigneten Dispergiermittel, beispielsweise Wasser, zugesetzt werden.

Wenn die wasserunlöslichen Metallphosphate in Form von Dispersionen eingesetzt und aufgesprüht werden, dann werden sie bevorzugt als wässrige Dispersionen eingesetzt, und es wird bevorzugt noch zusätzlich ein Entstaubungsmittel zur Fixierung des Additivs auf der Oberfläche des wasserabsorbierenden Polymers aufgebracht. Das Aufbringen des Entstaubungsmittels und der Dispersion erfolgt bevorzugt gemeinsam mit der Nachvernetzungslösung und kann gegebenenfalls aus einer gemeinsamen Lösung oder aus mehreren getrennten Lösungen über separate Düsensysteme zeitgleich oder zeitlich versetzt erfolgen. Bevorzugte Entstaubungsmittel sind dendritische Polymere, hochverzweigte Polymere, wie Polyglyzerine, Polyethylenglykole, Polypropylenglykole, statistische oder Block-Copolymere von Ethylenoxid und Propylenoxid. Weitere zu diesem Zweck geeignete Entstaubungsmittel sind die Polyethoxylate oder Polypropoxylate von Polyhydroxyverbindungen, wie Glyzerin, Sorbitol, Trimethylolpropan, Trimethylolethan und Pentaerythrit. Beispiele hierfür sind n-fach ethxoxyliertes Trimethylolpropan oder Glycerol, wobei n eine ganze Zahl zwischen 1 und 100 darstellt. Weitere Beispiele sind Block-Copolymere, wie insgesamt n-fach ethoxyliertes und dann m-fach propoxyliertes Trimethylolpropan oder Glyzerin, wobei n eine ganze Zahl zwischen 1 und 40 und m eine ganze Zahl zwischen 1 und 40 darstellt. Die Reihenfolge der Blöcke kann auch umgekehrt sein.

Die wasserunlöslichen Metallphosphate weisen eine mittlere Partikelgröße von üblicherweise weniger als 400 $\mu$m, vorzugsweise weniger als 100 $\mu$, bevorzugt weniger als 50 $\mu$m, besonders bevorzugt von weniger als 10 $\mu$m, auf, ganz besonders bevorzugt ist der Partikelgrößenbereich von 2 bis 7 $\mu$m.

Es ist aber auch möglich die wasserunlöslichen Metallphosphate erst auf der Oberfläche der wasserabsorbierenden Polymerpartikel zu erzeugen. Dazu werden Lösungen von Phosphorsäure oder löslicher Phosphate und Lösungen löslicher Metallsalze getrennt aufgesprüht, wobei sich das wasserunlösliche Metallphosphat auf der Partikeloberfläche bildet und abscheidet.

Die Beschichtung mit dem wasserunlöslichen Metallphosphat kann vor, während oder nach dem Nachvernetzungsschritt durchgeführt werden.

Die wasserabsorbierenden Polymerpartikel B werden vorzugsweise mit wasserunlöslichen Metallphosphaten beschichtet.

Die wasserabsorbierenden Polymerpartikel B weisen einen höheren Gehalt an Polymerpartikeln mit einer Partikelgröße von weniger als 300 $\mu$m auf. Die Flüssigkeitsweiterleitung (SFC) nimmt mit der Partikelgröße ab. Durch die Beschichtung mit wasserunlöslichen Metallphosphaten kann die Flüssigkeitsweiterleitung (SFC) erhöht werden, wobei wasserunlösliche Metallphosphate anderen möglichen anorganischen Zusatzstoffen deutlich überlegen sind. Da sich die Flüssigkeitsweiterleitung (SFC) und die Zentrifugenretentionskapazität (CRC) gegenseitig beeinflussen, kann bei Zusatz wasserunlöslicher Metallphosphate auch alternativ bei gleichbleibender Flüssigkeitsweiterleitung (SFC) die Zentrifugenretentionskapazität (CRC) erhöht werden.

Optional können aber alle bekannten Beschichtungen, wie filmbildende Polymere, Dendrimere, polykationische Polymere (wie Polyvinylamin, Polyethylenimin oder Polyallylamin), wasserunlösliche polyvalente Metallsalze, wie Kalziumsulfat, wasserlösliche polyvalente Metallsalze, wie Aluminiumsulfat, Kalzium- oder Magnesiumsalze, oder wasserlösliche Zirkoniumsalze, oder hydrophile anorganische Partikel, wie Tonminerale, pyrogene Kieselsäure, Aluminiumoxid und Magnesiumoxid, zusätzlich aufgebracht werden. Dadurch können zusätzliche Effekte, beispielsweise eine verringerte Verbackungsneigung, verbesserte Verarbeitungseigenschaften oder eine weiter gesteigerte Flüssigkeitsleitfähigkeit (SFC) erreicht werden. Wenn die Additive in Form von Dispersionen eingesetzt und aufgesprüht werden, dann

werden sie bevorzugt als wässrige Dispersionen eingesetzt, und es wird bevorzugt noch zusätzlich ein Entstaubungsmittel zur Fixierung des Additivs auf der Oberfläche des wasserabsorbierenden Polymers aufgebracht.

Nach dem erfindungsgemäßen Verfahren sind wasserabsorbierende Polymerpartikel mit guten Flüssigkeitstransporteigenschaften und einer guten Absorptionswirkung auf einfache Weise erhältlich. Beispielsweise ist es nicht erforderlich das Grundpolymer vor der Nachvernetzung mit Agglomeraten, wie mindestens 10 Gew.-% Agglomeraten aus Grundpolymerpartikeln, abzumischen.

Ebenso ist es unnötig bei der Herstellung der Grundpolymere Alkalisilikate zur zumindest teilweisen Neutralisation zu verwenden.

Eine Nachbehandlung des Grundpolymers mit polyvalenten Metallionen, wie Aluminumionen als Aluminiumsulfatlösung, ist ebenfalls nicht erforderlich.

Der Zusatz von Feststoffpulvern, insbesondere wasserlöslicher polyvalenter Metallsalze, beispielsweise Aluminiumsalze, wie Aluminiumsulfat, Kaliumalaun, Aluminiumnitrat, Aluminiumchlorid oder Natriumalaun, ist unnötig, insbesondere da die Salzpartikel nur als Abstandshalter zwischen den Polymerpartikein wirken und so kurzfristig die Flüssigkeitsweiterleitung erhöhen, bevor sie sich auflösen. Derartige Abstandshalter haben keinen positiven Einfluss auf die Porosität und damit die Dochtabsorption der wasserabsorbierenden Polymerpartikel.

Der Zusatz hydrophiler anorganischer Partikel, beispielsweise Tonminerale und pyrogene Kieselsäure, Aluminiumoxid und Magnesiumoxid, führt ebenfalls nur zu einer erhöhten Flüssigkeitsweiterleitung (SFC) ohne die Dochtabsorption zu verbessern.

Dies ist ein besonderer Vorteil des erfindungsgemäßen Verfahrens, da zusätzliche Vorrichtungen, beispielsweise zum Aufbringen von Beschichtungen, entfallen können.

Die erfindungsgemäßen Polymere zeichnen sich durch eine hohe Dochtabsorption aus. Diese kann mit dem weiter unten beschriebenen Dochtabsorptionstest bestimmt werden. Sie drückt sich durch eine hohe anfängliche Absorption sowie hohe Flüssigkeitsaufnahme nach 60 und 240 Minuten aus. Besonders gute superabsorbierende Polymere zeichnen sich darüber hinaus dadurch aus, dass auch nach über 150 Minuten noch eine weitere Flüssigkeitsaufnahme stattfindet.

Im drucklosen Dochtabsorptionstest beträgt die Menge 0,9 gew.-%iger Kochsalzlösung, die die erfindungsgemäßen Polymere innerhalb von 60 Minuten aufnehmen vorzugsweise mindestens 250 g, besonders bevorzugt mindestens 300 g, und die Menge 0,9 gew.-%iger Kochsalzlösung, die die erfindungsgemäßen Polymere innerhalb von 240 Minuten aufnehmen vorzugsweise mindestens 300 g, besonders bevorzugt mindestens 350 g.

Wird der Dochtabsorptionstest bei einem Druck von 0,3 psi (2070 Pa) durchgeführt, so beträgt die Menge 0,9 gew.-%iger Kochsalzlösung, die die erfindungsgemäßen Polymere innerhalb von 60 Minuten aufnehmen vorzugsweise mindestens 100 g, besonders bevorzugt mindestens 150 g, und die Menge 0,9 gew.-%iger Kochsalzlösung, die die erfindungsgemäßen Polymere innerhalb von 240 Minuten aufnehmen vorzugsweise mindestens 115 g, besonders bevorzugt mindestens 125 g.

Wird der Dochtabsorptionstest bei einem Druck von 0,7 psi (4830 Pa) durchgeführt, so beträgt die Menge 0,9 gew.-%iger Kochsalzlösung, die die erfindungsgemäßen Polymere innerhalb von 60 Minuten aufnehmen vorzugsweise mindestens 50 g, bevorzugt mindestens 60 g, besonders bevorzugt mindestens 70 g.

Die erfindungsgemäßen Polymere zeichnen sich weiterhin dadurch aus, dass die Dochtabsorption und die Flüssigkeitsleitfähigkeit beide so optimiert werden, dass das Produkt dieser beiden Kenngrößen für den Flüssigkeitstransport optimiert wird. Dieses Produkt aus Flüssigkeitsweiterleitung (SFC) und Dochtabsorption nach 60 min ($DA_{60}$) multipliziert mit $10^7$ wird als Transportwert bezeichnet (TW) und gemäß der unten angegebenen Formel berechnet.

Dabei werden Zentrifugenretentionskapazität (CRC) und Flüssigkeitsweiterleitung (SFC) über den Neutralisationsgrad des Grundpolymers sowie Zentrifugenretentionskapazität (CRC) und Transportwert (TW) über die Reaktionsbedingungen bei der Nachvernetzung optimiert. Insbesondere gilt, dass gering nachvernetzte Polymerpartikel allgemein eine hohe Zentrifugenretentionskapazität (CRC) und einen niedrigen Transportwert (TW) sowie hoch nachvernetzte Polymerpartikel allgemein eine niedrige Zentrifugenretentionskapazität (CRC) und einen hohen Transportwert (TW) aufweisen, wobei das Ausmaß der Nachvernetzung auch über die Reaktionstemperatur und die Reaktionszeit bestimmt wird.

Weiterhin sind die erfindungsgemäßen wasserabsorbierenden Polymerpartikel weitgehend frei von Verbindungen, die insbesondere während des Gebrauchs, zu unangenehmen Gerüchen führen.

Die erfindungsgemäßen wasserabsorbierenden Polymerpartikel sind sehr weiß, was insbesondere in ultradünnen Windeln mit einem hohen Anteil an wasserabsorbierenden Polymerpartikeln notwendig ist. Bei ultradünnen Windeln sind bereits geringe Farbvariationen durch die dünne Deckschicht zu erkennen, die von den Kunden nicht akzeptiert werden.

Ein weiterer Gegenstand der vorliegenden Erfindung sind Hygieneartikel, enthaltend erfindungsgemäße wasserabsorbierende Polymerpartikel, vorzugsweise ultradünne Windeln, enthaltend eine absorbierende Schicht bestehend aus 50 bis 100 Gew.-%, vorzugsweise 60 bis 100 Gew.-%, bevorzugt 70 bis 100 Gew.-%, besonders bevorzugt 80 bis 100 Gew.-%, ganz besonders bevorzugt 90 bis 100 Gew.-%, erfindungsgemäßer wasserabsorbierender Polymerpartikel,

wobei die Umhüllung der absorbierenden Schicht selbstverständlich nicht berücksichtigt ist.

Ganz besonders vorteilhaft sind die erfindungsgemäßen wasserabsorbierenden Polymerpartikel auch zur Herstellung von Laminaten und Kompositstrukturen, wie sie beispielsweise in der US-A 2003/0181115 sowie der US-A 2004/0019342 beschrieben sind, geeignet. Zusätzlich zu den in beiden Schriften zur Herstellung solcher neuer absorbierender Strukturen beschriebenen Schmelzklebern und insbesondere den in der US-A 2003/0181115 beschriebenen Fasern aus Schmelzklebern, an die die wasserabsorbierenden Polymerpartikel gebunden sind, eignen sich die erfindungsgemäßen wasserabsorbierenden Polymerpartikel auch zur Herstellung von vollkommen analogen Strukturen unter Verwendung von UV-vernetzbaren Schmelzklebern, welche beispielsweise als AC-Resin® (BASF AG, DE) vertrieben werden. Diese UV-vernetzbaren Schmelzkleber haben den Vorteil bereits bei 120 bis 140°C verarbeitbar zu sein, daher sind sie mit vielen thermoplastischen Substraten besser kompatibel. Ein weiterer wesentlicher Vorteil besteht darin, dass UV-vernetzbare Schmelzkleber toxikologisch sehr unbedenklich sind und auch keine Ausdünstungen in den Hygieneartikeln verursachen. Ein ganz wesentlicher Vorteil, im Zusammenhang mit den erfindungsgemäßen wasserabsorbierenden Polymerpartikeln, ist die Eigenschaft der UV-vernetzbaren Schmelzkleber während der Verarbeitung und Vernetzung nicht zur Vergilbung zu neigen. Dies ist insbesondere von Vorteil, wenn ultradünne oder teilweise transparente Hygieneartikel hergestellt werden sollen. Die Kombination der erfindungsgemäßen wasserabsorbierenden Polymerpartikel mit UV-vernetzbaren Schmelzklebern ist daher besonders vorteilhaft. Geeignete UV-vernetzbare Schmelzkleber sind beispielsweise beschrieben in EP-A 377 199, EP-A 445 641, US 5,026,806, EP-A 655 465 und EP-A 377 191.

Zur Bestimmung der Güte der Nachvernetzung werden die getrockneten wasserabsorbierenden Polymerpartikel mit den nachfolgend beschrieben Testmethoden geprüft.

Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymerpartikel werden vor der Messung gut durchmischt.

Zentrifugenretentionskapazität (CRC Centrifuge Retention Capacity)

Die Zentrifugenretentionskapazität wird gemäß der von der EDANA (European Disposables and Nonwovens Association) empfohlenen Testmethode Nr. 441.2-02 "Centrifuge retention capacity" bestimmt, jedoch wird abweichend davon für jedes Beispiel die tatsächliche Probe mit der dort angegegebenen Partikelgrößenverteilung gemessen.

Absorption unter Druck (AUL Absorbency Under Load)

Die Absorption unter Druck wird gemäß der von der EDANA (European Disposables and Nonwovens Association) empfohlenen Testmethode Nr. 442.2-02 " Absorption under pressure" bestimmt, jedoch wird abweichend davon für jedes Beispiel die tatsächliche Probe mit der dort angegegebenen Partikelgrößenverteilung gemessen.

Flüssigkeitsweiterleitung (SFC Saline Flow Conductivity)

Die Flüssigkeitsweiterleitung einer gequollenen Gelschicht unter Druckbelastung von 0,3 psi (2070 Pa) wird, wie in EP-A 640 330 beschrieben, als Gel-Layer-Permeability einer gequollenen Gelschicht aus wasserabsorbierenden Polymerpartikein bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP-A 640 330. Der Durchfluss wird automatisch erfasst.

Die Flüssigkeitsweiterleitung (SFC) wird wie folgt berechnet:

$$\mathrm{SFC}\ [\mathrm{cm^3 s/g}] = (\mathrm{Fg}(t=0) \times \mathrm{L0})/(\mathrm{d} \times \mathrm{A} \times \mathrm{WP}),$$

wobei Fg(t=0) der Durchfluss an NaCl-Lösung in g/s ist, der anhand einer linearen Regressionsanalyse der Daten Fg (t) der Durchflussbestimmungen durch Extrapolation gegen t=0 erhalten wird, L0 die Dicke der Gelschicht in cm, d die Dichte der NaCl-Lösung in g/$cm^3$, A die Fläche der Gelschicht in $cm^2$ und WP der hydrostatische Druck über der

Gelschicht in dyn/$cm^2$ darstellt.

Extrahierbare 16h

Der Gehalt an extrahierbaren Bestandteilen der wasserabsorbierenden Polymerpartikel wird gemäß der von der EDANA (European Disposables and Nonwovens Association) empfohlenen Testmethode Nr. 470.2-02 "Determination of extractable polymer content by potentiometric titration" bestimmt.

pH-Wert

Der pH-Wert der wasserabsorbierenden Polymerpartikel wird gemäß der von der EDANA (European Disposables and Nonwovens Association) empfohlenen Testmethode Nr. 400.2-02 "Determination of pH" bestimmt.

Free Swell Rate (FSR)

Zur Bestimmung der Quellgeschwindigkeit werden 1,00 g (= W1) der trockenen wasserabsorbierenden Polymerpartikel in ein 25 ml Becherglas eingewogen und gleichmässig auf dessen Boden verteilt. Dann werden 20 ml einer 0,9 gew.-%igen Kochsalzlösung mittels eines Dispensers in ein zweites Becherglas dosiert und der Inhalt dieses Glases wird dem ersten zügig hinzugefügt und eine Stopuhr gestartet. Sobald der letzte Tropfen Salzlösung absorbiert wird, was man am Verschwinden der Reflexion auf der Flüssigkeitsoberfläche erkennt, wird die Stopuhr angehalten. Die genaue Flüssigkeitsmenge, die aus dem zweiten Becherglas ausgegossen und durch das Polymer im ersten Becherglas absorbiert wurde, wird durch Rückwägung des zweiten Becherglases genau bestimmt (=W2). Die für die Absorption benötigte Zeitspanne, die mit der Stopuhr gemessen wurde, wird als t bezeichnet. Das Verschwinden des letzten Flüssigkeitstropfens auf der Oberfläche wird als Zeitpunkt t bestimmt.

Daraus errechnet sich die Quellgeschwindigkeit (FSR) wie folgt:

$$FSR\ [g/gs] = W2/(W1xt)$$

Wenn der Feuchtegehalt des hydrogelbildenden Polymeren jedoch mehr als 3 Gew.-% beträgt, so ist das Gewicht W1 um diesen Feuchtegehalt zu korrigieren.

Oberflächenspannung des wässrigen Extraktes

Es werden 0,50 g der wasserabsorbierenden Polymerpartikel in eine kleines Becherglas eingewogen und mit 40 ml einer 0,9 Gew.%-igen Salzlösung versetzt. Der Inhalt des Becherglases wird 3 Minuten bei 500 U/min mit einem Magnetrührstab gerührt, dann lässt man 2 Minuten absitzen. Schliesslich wird die Oberflächenspannung der überstehenden wässrigen Phase mit einem Digital-Tensiometer K10-ST oder vergleichbarem Gerät mit Platinplatte gemessen (Fa. Kruess). Die Messung wird bei einer Temperatur von 23°C durchgeführt.

Feuchtegehalt des Hydrogels

Der Wassergehalt der wasserabsorbierenden Polymerpartikel wird gemäß der von der EDANA (European Disposables and Nonwovens Association) empfohlenen Testmethode Nr. 430.2-02 "Moisture content" bestimmt.

Dochtabsorption

Versuchsaufbau:

Aufbau der Apparatur wie in Fig 1a (Seitenansicht) und Fig 1b (Aufsicht) dargestellt:

In einen zylindrischen 1.000 ml Tropftrichter (DIN 12566 graduiert, mit Kegelschliffhülse NS 29/32, Hahn mit Hohlglasküken und Ablaufrohr aus Duranglas, Teilung: 20 ml, Höhe 39,5 cm, Hersteller: Heico) mit Zweihals-Aufsatz aus Duranglas, mit Kegelschliffen NS 19/ 26 und NS 29 / 32 (Höhe 11 cm) werden 1.000 ml 0,9 %ige Kochsalzlösung gefüllt. Dann wird ein Glasrohr (Länge 47 cm, Innendurchmesser: 1,5 mm, Außendurchmesser: 4 mm) möglichst tief in den Tropftrichter eingeführt. Das Glasrohr wird mit einem durchbohrten Gummistopfen (oder einer Schraubdichtung) auf der einen Öffnung des Zweihals-Aufsatzes gegen die Außenatmosphäre abgeschlossen.

Auf eine Hebebühne wird eine an einen Computer angeschlossene Waage gestellt. Auf die Waage wird dann eine Plexiglasplatte (Fläche: 12 x 12 $cm^2$, Höhe: 1,5 cm), die mit einem Plexiglaszylinder (Höhe: 13,5 cm) verschweißt ist, gestellt. In die Plexiglasplatte ist eine P 0 Glasfritte mit einem Durchmesser von 7 cm und einer Höhe von 0,45 cm flüssigkeitsdicht eingebettet, d.h. die Flüssigkeit tritt durch die Poren der Fritte aus und nicht über den Rand zwischen Plexiglasplatte und Fritte. Durch den Außenmantel des Plexiglaszylinders ist ein Plexiglasrohr mit Krümmung in Mitte des Plexiglaszylinders angesetzt, um den Flüssigkeitstransport zu gewährleisten. Dieses Rohr hat den gleichen Durchmesser wie der Auslauf des Tropftrichters.

Das Flüssigkeitsrohr wird nun mit einem flexiblen Schlauch (Länge 37 cm, Außendurchmesser 1,0 cm, Innendurchmesser 0,7 cm) mit dem Tropftrichter in einer Weise verbunden, dass durch die Verbindung kein Druck auf die Waage ausgeübt wird.

Mit der Hebebühne wird die Oberseite der Fritte nun auf das Niveau des unteren Endes des Glasrohres gebracht, so dass während der Messung ein stetig druckloser (Atmosphärendruck) Flüssigkeitsfluss aus dem Tropftrichter zur Messapparatur gewährleistet ist. Die Oberseite der Fritte wird dabei so justiert, dass ihre Oberfläche feucht ist, jedoch kein Wasserfilm auf der Fritte steht. (Außerdem ist darauf zu achten, dass bei der Justierung keine Flüssigkeit im Glasrohr steht. Ist dies der Fall, ist die Fritte falsch justiert. Ferner darf sich bei geöffnetem Hahn des Tropftrichters auch 5 Minuten nach beendeter Justierung kein durchgehender Flüssigkeitsfilm auf der Fritte bilden und das Gewicht der Waage muß in dieser Zeit unverändert bleiben. Sollten auf der Unterseite der Fritte Gasblasen zu erkennen sein, so werden diese z.B. durch Anwendung einer Absaugung entfernt). Während der Justierung ist der Hahn des Tropftrichters geöffnet; sobald die Justierung abgeschlossen ist, wird er geschlossen.

Die einwandfreie Funktionsweise wird vor jedem Versuch überprüft. Vor jedem Versuch wird die Flüssigkeit im Tropftrichter auf 1.000 ml aufgefüllt.

In den trockenen Plexiglaszylinder werden 70,0 $\pm$ 0,1 g wasserabsorbierende Polymerpartikel eingewogen und die Oberfläche der wasserabsorbierenden Polymerpartikel wird geglättet. Die Füllhöhe beträgt ca. 3 cm. Der Plexiglaszylinder wird auf die (feuchte) Fritte gestellt und die Waage wird tariert. Dann wird der Hahn des Tropftrichters geöffnet und die Messung gestartet.

Durch die hohe Gewichtsbelastung kann es vorkommen, dass die Spannung des Drahtgewebes im Lauf der Zeit (nach vielen Messungen) nachlässt und sich das Gewebe während der Messung nach außen verbeult. In diesem Fall bewegt sich der Plexiglaszylinder mit dem Polymer während der Messung aus der Senkrechten hinaus. Es ist dann kein optimaler Kontakt mehr zwischen Drahtgewebe und Glasfritte vorhanden, mit der Folge, dass die Flüssigkeitsaufnahme absinkt. In diesem Fall ist ein Austausch des Drahtgewebes erforderlich.

Versuchsdurchführung:

Wenn die Apparatur vollständig zusammengebaut ist, wird der Hahn des Tropftrichters geöffnet und die elektronische Datenaufzeichnung gestartet. Es wird die Zunahme des Gewichts auf der Waage in Abhängigkeit von der Zeit registriert. Dies gibt dann an wieviel Salzlösung zu einem bestimmten Zeitpunkt von der quellenden Gelsäule der wasserabsorbierenden Polymerpartikel aufgenommen worden ist. Man lässt die Gelsäule 60 bzw. 240 Minuten lang quellen. Die Daten werden alle 10 Sekunden automatisch erfasst. Der Test kann drucklos durchgeführt werden, das bedeutet es liegt kein Gewicht auf der Gelsäule während des Quellvorgangs. Wahlweise kann zur besseren Differenzierung aber auch eine Quellung unter Druck erfolgen, wobei man die oben unter Absorption unter Druck (AUL) bezeichneten Gewichte in die Apparatur einsetzen kann. Übliche Gewichtsbelastungen sind 0.3 psi (2,07 kPa) und 0.7 psi (4,83 kPa). Die Messung wird bevorzugt drucklos sowie bei 0.3 psi und 0.7 psi über einen Zeitraum von jeweils 60 oder 240 Minuten pro Probe durchgeführt. Die Absorptionsdaten beziehen sich jeweils auf die von der 70 g-Probe insgesamt aufgenommene Menge der Salzlösung.

Die innerhalb einer bestimmten Zeit von der 70 g-Probe insgesamt aufgenommene Menge Salzlösung (in Gramm) wird als Dochtabsorption bezeichnet und wie folgt gekennzeichnet: $DA_{nn}$, wobei der tiefgestellte Index nn die Absorptionszeit in Minuten kennzeichnet, beispielsweise bedeutet $DA_{60}$ die drucklose Absorption nach 60 Minuten. Wenn ein Druck während des Quellvorganges wirkt, dann wird dieser in Klammern dargestellt, beispielsweise bedeutet $DA_{240}$(0.3 psi) die Absorption unter einem Druck von 0.3 psi nach 240 Minuten.

Transportwert (TW)

Das Produkt aus Dochtabsorption und Flüssigkeitsleitfähigkeit (SFC) kennzeichnet die Fähigkeit der wasserabsorbierenden Polymerpartikel zum Transport von Flüssigkeiten beim Quellen oder durch die gequollene Gelschicht. Dieses Produkt aus SFC und $DA_{60}$ (drucklose Dochtabsorption nach 60 min) wird als Transportwert bezeichnet (TW) und wie folgt berechnet:

$$TW\,[cm^3 s] = SFC\,[cm^3 s/g] \times DA_{60}\,[g] \times 10^7$$

Geruchstest

Zur Beurteilung des Geruchs der angequollenen wasserabsorbierenden Polymerpartikel werden 2,0 g trockene Polymerpartikel in ein 50 ml Becherglas eingewogen. Dann werden 20 g 0,9 Gew-%ige Kochsalzlösung bei 23°C zugegeben. Das Glas mit den quellenden wasserabsorbierenden Polymerpartikeln wird mit Parafilm abgedeckt und 3 Minuten stehen gelassen. Danach wird der Film entfernt und der Geruch kann beurteilt werden. Jede Probe wird dabei von mindestens 3 Versuchspersonen geprüft, wobei jeweils eine separate Probe hergestellt wird.

CIE-Farbzahl (L a b)

Die Farbmessung wurde entsprechend dem CIELAB-Verfahren (Hunterlab, Band 8, Jahrgang 1996, Heft 7, Seiten 1 bis 4) durchgeführt. Dabei werden die Farben in über die Koordinaten L*, a* und b* eines dreidimensionalen Systems beschrieben. Dabei gibt L* die Helligkeit an, wobei L* = 0 schwarz und L* = 100 weiß bedeutet. Die Werte für a* und b* geben die Position der Farbe auf den Farbachsen rot/grün bzw. gelb/blau an, wobei +a* für rot, -a* für grün, +b* für gelb und -b* für blau steht.
Die Farbmessung entspricht dem Dreibereichsverfahren nach DIN 5033-6.
Der Hunter 60-Wert ist ein Maß für den Weißegrad von Oberflächen und ist definiert als L*-3b*, d.h., der Wert ist umso niedriger, je dunkler und je gelbstichiger eine Farbe ist.
Verwendet wurde ein Hunterlab LS 5100 Colorimeter.
Die EDANA-Testmethoden sind beispielsweise erhältlich bei der European Disposables and Nonwovens Association, Avenue Eugène Plasky 157, B-1030 Brüssel, Belgien.

Beispiele

Beispiel 1 - Herstellung Grundpolymer

In einen Lödige-Pflugscharkneter Typ VT 5R-MK (5 l Volumen) wurden 294 g entionisiertes Wasser, 306,6 g Acrylsäure, 1993,0 g einer 37,3 gew.%igen Natriumacrylatlösung (100 mol% neutralisiert) sowie 3,50 g des Vernetzers 18-fach ethoxyliertes Trimethylolpropantriacrylat vorgelegt und unter Durchperlen von Stickstoff 20 Minuten inertisiert. Dann wurde durch Zusatz (verdünnte wässrige Lösungen) von 2,453 g Natriumpersulfat gelöst in 13,9 g Wasser, 0,053 g Ascorbinsäure gelöst in 10,46 g Wasser sowie 0,146 g Wasserstoffperoxid (30 gew.-%ig) gelöst in 1,31 g Wasser bei ca. 20 °C gestartet. Nach dem Start wurde die Temperatur des Heizmantels der Reaktionstemperatur im Reaktor mittels Regelung nachgeführt. Das letztlich erhaltene krümelige Gel wurde dann bei 160°C ca. 3 Stunden im Umlufttrockenschrank getrocknet.
Das getrocknete Grundpolymer wurde gemahlen und auf 300 bis 600 μm abgesiebt.
Die Eigenschaften des Polymers (300 bis 600 μm) waren wie folgt:

CRC = 32 g/g
AUL 0.3 psi = 26 g/g
Extrahierbare 16h = 9,8 Gew.-%
pH = 5,8

Beispiel 2a

20 g des Polymers aus Beispiel 1 wurden in einem Waring-Labormischer vorgelegt, der mit einem Aufsatz mit stumpfen Mischklingen ausgerüstet war. Bei mittlerer Drehzahl wurden dann mittels einer Injektionsspritze langsam durch ein Loch im Deckel des Mischaufsatzes genau 1,00 g der Nachvernetzungslösung langsam und unter Rühren zugefügt, um das Polymer möglichst gleichmäßig zu benetzen.
Die Nachvernetzungslösung hatte folgende Zusammensetzung: 0,02 g 2-Oxazolidon, 0,01 g Sorbitanmonococoat, 0,97 g Wasser.
Das befeuchtete Polymer wurde durch Rühren homogenisiert und dann auf einem Uhrglas im Umlufttrockenschrank für 90 Minuten bei 175°C getempert. Schließlich wurde durch ein 600 μm Sieb gesiebt, um Klumpen zu entfernen.
Das Polymer hatte folgende Eigenschaften:

CRC = 26 g/g
AUL 0.7 psi = 23 g/g
SFC = 129 $\times$ $10^{-7}$ $cm^3$ s $g^{-1}$
FSR = 0,16 g $g^{-1}$ $s^{-1}$

Beispiel 2b

20 g des Polymers aus Beispiel 1 wurden in einem Waring-Labormischer vorgelegt, der mit einem Aufsatz mit stumpfen Mischklingen ausgerüstet war. Bei mittlerer Drehzahl wurden dann mittels einer Injektionsspritze langsam durch ein Loch im Deckel des Mischaufsatzes genau 1,00 g der Nachvernetzungslösung langsam und unter Rühren zugefügt, um das Polymer möglichst gleichmäßig zu benetzen.
Die Nachvernetzungslösung hatte folgende Zusammensetzung: 0,020 g N-Hydroxyethyl-2-Oxazolidon, 0,005 g Sorbitanmonococoat, 0,975 g Wasser.
Das befeuchtete Polymer wurde durch Rühren homogenisiert und dann auf einem Uhrglas im Umlufttrockenschrank für 90 Minuten bei 175°C getempert. Schließlich wurde durch ein 600 µm Sieb gesiebt , um Klumpen zu entfernen.
Das Polymer hatte folgende Eigenschaften:

CRC = 26,1 g/g
AUL 0.7 psi = 23,5 g/g
SFC = 120 $\times$ $10^{-7}$ $cm^3$ s $g^{-1}$
FSR = 0,16 g $g^{-1}$ $s^{-1}$

Beispiel 2c

20 g des Polymers aus Beispiel 1 wurden in einem Waring-Labormischer vorgelegt, der mit einem Aufsatz mit stumpfen Mischklingen ausgerüstet war. Bei mittlerer Drehzahl wurden dann mittels einer Injektionsspritze langsam durch ein Loch im Deckel des Mischaufsatzes genau 1,00 g der Nachvernetzungslösung langsam und unter Rühren zugefügt, um das Polymer möglichst gleichmäßig zu benetzen.
Die Nachvernetzungslösung hatte folgende Zusammensetzung: 0,020 g 2-Oxazolidon, 0,020 g 1,2-Propandiol, 0,010 g Sorbitanmonococoat, 0,95 g Wasser.
Das befeuchtete Polymer wurde durch Rühren homogenisiert und dann auf einem Uhrglas im Umlufttrockenschrank für 90 Minuten bei 175°C getempert. Schließlich wurde durch ein 600 µm Sieb gesiebt, um Klumpen zu entfernen.
Das Polymer hatte folgende Eigenschaften:

CRC = 25,8 g/g
AUL 0.7 psi = 22,8 g/g
SFC = 140 $\times$ $10^{-7}$ $cm^3$ s $g^{-1}$
FSR = 0,14 g $g^{-1}$ $s^{-1}$

Beispiel 2d

20 g des Polymers aus Beispiel 1 wurden in einem Waring-Labormischer vorgelegt, der mit einem Aufsatz mit stumpfen Mischklingen ausgerüstet war. Bei mittlerer Drehzahl wurden dann mittels einer Injektionsspritze langsam durch ein Loch im Deckel des Mischaufsatzes genau 1,00 g der Nachvernetzungslösung langsam und unter Rühren zugefügt, um das Polymer möglichst gleichmäßig zu benetzen.
Die Nachvernetzungslösung hatte folgende Zusammensetzung: 0,020 g 2-Oxazolidon, 0,29 g Isopropanol, 0,69 g Wasser.
Das befeuchtete Polymer wurde durch Rühren homogenisiert und dann auf einem Uhrglas im Umlufttrockenschrank für 90 Minuten bei 175°C getempert. Schließlich wurde durch ein 600 µm Sieb gesiebt, um Klumpen zu entfernen.
Das Polymer hatte folgende Eigenschaften:

CRC = 25,2 g/g
AUL 0.7 psi = 23,2 g/g
SFC = 119 $\times$ $10^{-7}$ $cm^3$ s $g^{-1}$
FSR = 0,14 g $g^{-1}$ $s^{-1}$

Beispiel 3

Herstellung von Grundpolymeren mit unterschiedlichem Neutralisationsgrad und Nachvernetzung mit 2-Oxazolidon.

Analog Beispiel 1 wurden in einem Lödige-Pflugscharkneter Typ VT 5R-MK (5 l Volumen) bei einem Feststoffgehalt von 35,5 Gew.-% und Neutralisationsgraden von 60 mol-%, 65 mol-%, 70 mol-%, 75 mol-%, und 85 mol-% und einem Vernetzereinsatz von 0,70 Gew.-% Polyethylenglykol-400-diacrylat (bezogen auf Acrylsäure) wasserabsorbierende Polymere hergestellt. Die Initiation erfolgte analog zu Beispiel 1 nach Vorlage der Einsatzstoffe. Es wurden diese unter Durchperlen von Stickstoff 20 Minuten inertisiert. Dann wurde durch Zusatz (verdünnte wässrige Lösungen) von 2,453 g Natriumpersulfat gelöst in 13,9 g Wasser, 0,053 g Ascorbinsäure gelöst in 10,46 g Wasser sowie 0,146 g Wasserstoffperoxid (30 gew.-%ig) gelöst in 1,31 g Wasser bei ca. 20°C gestartet. Nach dem Start wurde die Temperatur des Heizmantels der Reaktionstemperatur im Reaktor mittels Regelung nachgeführt. Das letztlich erhaltene krümelige Gel wurde dann bei 160°C ca. 3 Stunden im Umlufttrockenschrank getrocknet.

Das getrocknete Grundpolymer wurde gemahlen und auf 200 bis 850 μm abgesiebt.

20 g jedes dieser Polymere wurde in einem Waring-Labormischer vorgelegt, der mit einem Aufsatz mit stumpfen Mischklingen ausgerüstet ist. Bei mittlerer Drehzahl wurden dann mittels einer Injektionsspritze langsam durch ein Loch im Deckel des Mischaufsatzes genau 1,00 g der Nachvernetzungslösung langsam und unter Rühren zugefügt, um das Polymer möglichst gleichmäßig zu benetzen.

Die Zusammensetzung der Nachvernetzungslösung ist so definiert, dass bezogen auf das eingesetzte Grundpolymer 3,43 Gew.-% Wasser, 1,47 Gew.-% Isopropanol, und 0,10 Gew.-% Oxazolidon aufgebracht wurden. Das befeuchtete Polymer wurde durch Rühren homogenisiert und dann wurden die Polymere auf Uhrgläsern in Umlufttrockenschränken für 60 Minuten bei 175°C getempert. Schließlich wurde durch ein 850 μm Sieb gesiebt, um Klumpen zu entfernen.

Die Eigenschaften der so hergestellten Polymere sind in der folgenden Tabelle zusammengefasst.

Tab. 1: Abhängigkeit von CRC, AUL und SFC vom Neutralisationsgrad

| Neutralisationsgrad | pH-Wert des Polymers | CRC [g/g] | AUL 0.7 psi [g/g] | SFC [$\times 10^{-7}$ $cm^3$ s $g^{-1}$] |
|---|---|---|---|---|
| 85 mol-% | 6,32 | 29,1 | 25,0 | 22 |
| 75 mol-% | 5,97 | 29,2 | 26,8 | 45 |
| 70 mol-% | 5,79 | 29,0 | 26,8 | 57 |
| 65 mol-% | 5,65 | 27,5 | 26,5 | 70 |
| 60 mol-% | 5,49 | 25,5 | 25,0 | 80 |

Aus den Daten wird ersichtlich, dass bei zu hohem Neutralisationsgrad die Flüssigkeitsweiterleitung (SFC) stark abfällt, jedoch bei zu niedrigem Neutralisationsgrad die Zentrifugenretentionskapazität (CRC) stark beeinträchtigt wird.

Beispiele 5 bis 14

Das Herstellungsbeispiel für das Grundpolymer aus Beispiel 1 wurde wiederholt. Das getrocknete Grundpolymer wurde gemahlen und auf 300 bis 600 μm abgesiebt. Die Eigenschaften des Grundpolymers (300 bis 600 μm) waren wie folgt:

CRC = 32 g/g
AUL 0.3 psi = 24 g/g
Extrahierbare 16h = 8,2 Gew.-%
pH = 5,7

Die Nachvernetzung wurde völlig analog zu den Beispielen 2a bis 2d durchgeführt. Die folgende Tabelle gibt die Einsatzstoffmengen in Gew.-% bezogen auf eingesetztes Grundpolymer an sowie die Reaktionsbedingungen. Nachvernetzer 1 und gegebenenfalls Nachvernetzer 2 wurden zusammen mit 0,02 Gew.-% Sorbitanmonococoate (Deagglomerationsmittel, Emulsogen V 4345 der Clariant, DE)), bezogen auf eingesetztes Polymer, und gegebenenfalls dem Cosolvens gelöst und im Waring-Blender auf das Grundpolymer aufgesprüht. Anschließend wurde auf einem Uhrglas im Umluftofen bei der angegebenen Verweilzeit und Reaktionstemperatur getrocknet. Die Ergebnisse sind in Tabelle 2 zusammengefasst.

Tab. 2: Nachvernetzung eines Grundpolymers mit einem Neutralisationsgrad von 65 mol-%

| Bsp. | Nachvernetzer 1 | Nachvernetzer 2 | Wasser | Cosolvens | Sorbitan-monococoat | Verweilzeit | Reaktions-temperatur | CRC [g/g] | AUL 0.7psi [g/g] | SFC [$x10^{-7}cm^3 sg^{-1}$] | Geruchstest |
|---|---|---|---|---|---|---|---|---|---|---|---|
| 5 | 0,10 Gew.% Ethylenglycoldiglycidyiether | -/- | 3,88 Gew.% | -/- | 0,02 Gew.% | 60 min | 175˚C | 27,5 | 24,4 | 91 | |
| 6 | 0,12 Gew.% 2-Oxazolidinon | -/- | 3,86 Gew.% | -/- | 0,02 Gew.% | 60 min | 175˚C | 26,3 | 23,0 | 121 | |
| 7 | 0,12 Gew.% 2-Oxazolidinon | 0,12 Gew.% Propandiol-1,2 | 3,74 Gew.% | -/- | 0,02 Gew.% | 60 min | 175˚C | 27,2 | 23,5 | 207 | alkoholisch |
| 8 | 0,12 Gew.% Ethylencarbonat | -/- | 3,86 Gew.% | -/- | 0,02 Gew.% | 60 min | 175˚C | 27,0 | 23,8 | 129 | |
| 9 | 0,12 Gew.% N-Hydroxyethyl-2-Oxazolidinon | -/- | 3,86 Gew.% | -/- | 0,02 Gew.% | 60 min | 195˚C | 25,2 | 22,1 | 117 | |
| 10 | 0,12 Gew.% N-Hydroxyethyl-2-Oxazolidinon | 0,12 Gew.% Propandiol-1,2 | 3,74 Gew.% | -/- | 0,02 Gew.% | 90 min | 175˚C | 26,6 | 23,5 | 128 | alkoholisch |
| 11 | 0,60 Gew.% Propandiol-1,2 | -/- | 3,38 Gew % | -/- | 0,02 Gew.% | 60 min | 175˚C | 26,8 | 23,3 | 133 | alkoholisch |
| 12 | 0,60 Gew.% Propandiol-1,2 | -/- | 2,38 Gew % | 1,02Gew.% Isopropanol | 0,02 Gew.% | 60 min | 175˚C | 25,9 | 22,1 | 155 | alkoholisch |
| 13 | 0,24 Gew.% 5-Methyl-1-aza-4,6-dioxabicyclo[3.3.0]octan | -/- | 3,74 Gew % | -/- | 0,02 Gew.% | 60 min | 175˚C | 26,5 | 21,1 | 90 | |
| 14 | 0,24 Gew.% 1-Aza-4,6-dioxabicyclo [3.3.0]octan | -/- | 3,74 Gew.% | -/- | 0,02 Gew.% | 60 min | 175˚C | 26,6 | 21,8 | 92 | |

Beispiel 15 - Herstellung Grundpolymer:

Das Herstellungsbeispiel für Grundpolymer aus Beispiel 1 wurde wiederholt. Das getrocknete Grundpolymer wurde gemahlen und auf 300 bis 600 μm abgesiebt. Die Eigenschaften des Grundpolymers (300 bis 600 μm) waren wie folgt:

CRC = 33 g/g
AUL 0.3 psi = 23 g/g
Extrahierbare 16h = 7,8 Gew.-%
pH = 5,7

Beispiel 16 bis 36 - Oberflächennachvernetzung

Die Nachvernetzung wurde völlig analog zu den Beispielen 2a bis 2d durchgeführt. Die folgende Tabelle gibt die Einsatzstoffmengen des Vernetzers im Gemisch mit Wasser in Gew.-% bezogen auf eingesetztes Grundpolymer an. Zur Nachvernetzung wurden jeweils 20 g Grundpolymer aus Beispiel 15 mit der angegebenen Mischung, die noch 0,02 Gew.-% Sorbitanmonococoate (Deagglomerationsmittel Emulsogen V 4345, Clariant, DE), bezogen auf eingesetztes Polymer, enthielt, im Waring-Blender besprüht und vermischt. Anschliessend wurde auf einem Uhrglas im Umluftofen bei 175°C für 60 Minuten getrocknet. Die Ergebnisse sind in den Tabellen 3 und 4 zusammengefaßt.

Tab. 3: Nachvernetzung mit verschiedenen Nachvernetzern

| Beispiel | Nachvernetzer | Formel | Nachvernetzer Gew.% | Wasser Gew.% | CRC (g/g) | AUL 0.7 psi (g/g) | SFC ($x10^{-7}cm^3xs/g$) | CIE-Farbzahl L | a | b | Geruchstest |
|---|---|---|---|---|---|---|---|---|---|---|---|
| 16 | 1,2-Ethandiol | $HO(CH_2)_2OH$ | 1,32 | 2,68 | 24,5 | 21,4 | 175 | 90,1 | -0,7 | 57 | alkoholisch |
| 17 | 1,2-Propandiol | $CH_3CH(OH)CH_2OH$ | 1,32 | 2,68 | 25,0 | 21,2 | 186 | 90,1 | -0,7 | 5,6 | alkoholisch |
| 18 | 1,4-Butandiol | $HO(CH_2)_4OH$ | 1,32 | 2,68 | 26,2 | 21,4 | 189 | 90,4 | -0,7 | 5,2 | geruchsfrei |
| 19 | Trimethylolpropan Ethoxylat Polyol TP 70 (n=7) | $C_2H_5C[CH_2(OCH_2CH_2)_nOH]_3$ | 1,32 | 2,68 | 30,6 | 19,5 | 1 | 89,4 | -0,7 | 5,5 | geruchsfrei |
| 20 | Trimethylolpropan Propoxylat Polyol TS 30 (n=3) | $C_2H_5C[CH_2(OC_3H_6)_nOH]_3$ | 1,32 | 2,68 | 31,4 | 13,0 | 0 | 89,8 | -0,7 | 5,3 | geruchsfrei |
| 21 | 5-Ethyl-1,3-dioxan-5-methanol | 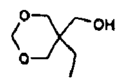 | 1,32 | 2,68 | 31,1 | 18,4 | 4 | 90,4 | -0,7 | 5.2 | geruchsfrei |
| 22 | Di (Trimethylolpropan) | $O[CH_2C(C_2H_5)(CH_2OH)_2]_2$ | 1,32 | 2,68 | 30,2 | 21,1 | 8 | 90,5 | -0,7 | 5,7 | fast geruchsfrei |
| 23 | 2,5-Hexandiol | $CH_3CH(OH)CH_2CH_2CH(OH)CH_3$ | 1,32 | 2,68 | 30,3 | 21,4 | 9 | 90,4 | -0,7 | 5,3 | stinkt aromatisch |
| 24 | 2-Methyl-2,4-Pentandiot | $CH_3CH(OH)CH_2C(CH_3)_2OH$ | 1,32 | 2,68 | 30,8 | 16,7 | 0 | 90,7 | -0,7 | 5,4 | stinkt aromatisch |
| 25 | 2,4-Dimethyl-2,4-Pentandiol | $HOC(CH_3)_2CH_2C(CH_3)_2OH$ | 1,32 | 2,68 | 30,7 | 14,2 | 0 | 90,5 | -0,7 | 5,4 | fast geruchsfrei |
| 26 | Neopentyl Glycol Propoxylat Polyol NS 20 (n=2) | $(CH_3)_2C[CH_2[OCH_2CH(CH_3)]_nOH]_2$ | 1,32 | 2,68 | 30,3 | 16,8 | 3 | 90,3 | -0,7 | 5,3 | schwach fruchtig |
| 27 | Trimethylolpropan Ethoxylat Polyol TP 30 (n=3) | $C_2H_5C[CH_2(OCH_2CH_2)_nOH]_3$ | 1,32 | 2,68 | 29,4 | 24,2 | 28 | 90,3 | -0,7 | 5,4 | schwach alkoholisch |

Tab. 4: Nachvernetzung mit verschiedenen bevorzugten Nachvernetzern

| Beispiel | Nachvernetzer | Formel | Nachvernetzer Gew.% | Wasser Gew.% | CRC (g/g) | AUL 0.7 psi (g/g) | SFC ($x10^{-7}cm^3xslg$) | CIE-Farbzahl L | a | b | Geruchstest |
|---|---|---|---|---|---|---|---|---|---|---|---|
| 28 | 1,3-Propandiol | $HO(CH_2)OH$ | 1,32 | 2,68 | 24,6 | 21,4 | 168 | 90,8 | -0,7 | 5,2 | geruchsfrei |
| 29 | 1,5-Pentandiol | $HO(CH_2)_5OH$ | 1,32 | 2,68 | 26,9 | 21,3 | 165 | 90,4 | -0,7 | 5,2 | geruchsfrei |
| 30 | 1,6-Hexandiol | $HO(CH_2)_6OH$ | 1,32 | 2,68 | 27,6 | 23,3 | 106 | 90,5 | -0,7 | 5,3 | geruchsfrei |
| 31 | 1,7-Heptandiol | $HO(CH_2)_7OH$ | 1,32 | 2,68 | 29,4 | 23,3 | 52 | 90,5 | -0,7 | 5,3 | geruchsfrei |
| 34 | 2-Methyl-1,3-Propandiol | $HOCH_2CH(CH_3)CH_2OH$ | 1,32 | 2,68 | 26,7 | 22,4 | 182 | 90,6 | -0,7 | 5,2 | geruchsfrei |
| 32 | 2,2-Oimethyl-1,3-Propandiol | $HOCH_2C(CH_{-3})_2CH_2OH$ | 1,32 | 2.68 | 27,8 | 23,8 | 89 | 90,6 | -0,7 | 5,2 | schwach fruchtig |
| 33 | Trimethylolpropan Ethoxylat Polyol TP 08 (n=0,8) | $C_2H_5C[CH_2(OCH_2CH_2)_nOH]_3$ | 1,32 | 2,68 | 27,8 | 22,4 | 93 | 90,5 | -0,7 | 5,2 | geruchsfrei |
| 35 | 3-Ethyl-3-Oxetanmethanoi | 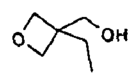 | 1,32 | 2,68 | 27,7 | 22,4 | 131 | 90,1 | -0,6 | 5,2 | geruchsfrei |
| 36 | 1,1,1-Tris (Hydroxymethyl) propan | $C_2H_5C(CH_2OH)_3$ | 1,32 | 2,68 | 27,4 | 22,1 | 111 | 90,7 | -0,7 | 5,2 | geruchsfrei |

In den Beispielen 16 bis 18 wurden zwar gute Nachvernetzungsergebnisse erzielt, die hergestellten wasserabsorbierenden Polymerpartikel setzten aber bei Kontakt mit Wasser entweder unangenehme Gerüche aus entstandenen Nebenprodukten, oder, im Fall von 1,4-Butandiol als Nachvernetzer, durch Zyklisierung unerwünschte flüchtige wenig riechende Nebenprodukte (Tetrahydrofuran) frei.

In den Beispielen 23 und 24 entstanden bei der Nachvernetzung stark riechende Nebenprodukte, die bei Quellung der wasserabsorbierenden Polymerpartikel in wässriger Lösung freigesetzt wurden und die Verwendung solcher Polymerpartikel in Hygieneartikeln ausschliessen.

Die Beispiele 19 bis 22 sowie 25 bis 27 sind Beispiele für wenig reaktive Nachvernetzer, die vorzugsweise als Cosolvens eingesetzt werden.

Beispiel 37 bis 57 - Oberflächennachvernetzung mit Reaktivvernetzer

Die Nachvernetzung wurde völlig analog zu den Beispielen 2a bis 2d durchgeführt. Die folgende Tabelle gibt die Einsatzstoffmengen des Vernetzers im Gemisch mit Wasser in Gew.-% bezogen auf eingesetztes Grundpolymer an. Zur Nachvernetzung wurden jeweils 20 g Grundpolymer aus Beispiel 15 mit der in der Tabelle angegebenen Mischung aus Vernetzer oder Cosolvens und 2-Oxazolidon, die noch zusätzlich Sorbitanmonococoate (Deagglomerationsmittel Emulsogen V 4345, Clariant, DE) in einer Menge von 0,02 Gew.-%, bezogen auf eingesetztes Polymer, enthielt, im Waring-Blender besprüht und vermischt. Anschließend wurde auf einem Uhrglas im Umluftofen bei 175°C für 60 Minuten getrocknet. Die Ergebnisse sind in den Tabellen 5 und 6 zusammengefaßt.

Tab. 5: Nachvernetzung mit verschiedenen wenig reaktiven Nachvernetzern als Cosolvens

| Bsp. | Nachvernetzer oder Cosolvens | Formel | Nachvernetzer Gew.% | Wasser Gew.% | 2-Oxazolidon Gew.% | CRC (g/g) | AUL 0.7 psi (g/g) | SFC ($x10^{-7}cm^3xs/g$) | CIE-Farbzahl L | a | b | Geruchstest |
|---|---|---|---|---|---|---|---|---|---|---|---|---|
| 37 | 1,2-Ethandiol | $HO(CH_2)_2OH$ | 1,34 | 2,67 | 0,10 | 24,6 | 21,4 | 173 | 90,2 | -0,8 | 6,5 | alkoholisch |
| 38 | 1,2-Propandiol | $CH_3CH(OH)CH_2OH$ | 1,34 | 2,67 | 0,10 | 24,6 | 21,3 | 161 | 90,2 | -0,8 | 7,2 | alkoholisch |
| 39 | 1,4-Butandiol | $HO(CH_2)_4OH$ | 1,34 | 2,67 | 0,10 | 25,9 | 21,9 | 122 | 90,4 | -0,7 | 5,4 | geruchsfrei |
| 40 | 2,5-Hexandiol | $CH_3CH(OH)CH_2CH_2CH(OH)CH_3$ | 1,34 | 2,67 | 0,10 | 27,7 | 23,1 | 143 | 90,5 | -0,7 | 5,5 | stinkt aromatisch |
| 41 | 2-Methyl-2,4-Pentandiol | $CH_3CH(OH)CH_2C(CH_3)_2OH$ | 1,34 | 2,67 | 0,10 | 28,0 | 23,5 | 146 | 90,5 | -0,7 | 5,6 | stinkt aromatisch |

Tab 6: Nachvernetzung mit verschiedenen bevorzugten wenig reaktiven Nachvernetzern als Cosolvens

| Bsp. | Nachvernetzer oder Cosolvens | Formel | Nachvernetzer Gew.% | Wasser Gew.% | 2-Oxazolidon Gew.% | CRC (g/g) | AUL 0.7 psi (g/g) | SFC ($x10^{-7}cm^3xs/g$) | CIE-Farbzahl L | a | b | Geruchstest |
|---|---|---|---|---|---|---|---|---|---|---|---|---|
| 42 | 1,3-Propandiol | $HO(CH_2)_3OH$ | 1,34 | 2,67 | 0,10 | 25,1 | 21,7 | 132 | 90,7 | -0,8 | 5,8 | geruchsfrei |
| 43 | 1,5-Pentandiol | $HO(CH_2)_5OH$ | 1,34 | 2,67 | 0,10 | 26,7 | 22,0 | 177 | 90,5 | -0,7 | 5,3 | geruchsfrei |
| 44 | 1,6-Hexandiol | $HO(CH_2)_6OH$ | 1,34 | 2,67 | 0,10 | 27,2 | 24,0 | 145 | 90,5 | -0,7 | 5,5 | geruchsfrei |
| 45 | 1,7-Heptandiol | $HO(CH_2)_7OH$ | 1,34 | 2,67 | 0,10 | 27,4 | 22,1 | 150 | 90,8 | -0,7 | 5,4 | geruchsfrei |
| 46 | 2,4-Dimethyl-2,4-Pentandiol | $HOC(CH_3)_2CH_2C(CH_3)_2OH$ | 1,34 | 2,67 | 0,10 | 27,5 | 21,7 | 138 | 90,5 | -0,7 | 5,4 | fast geruchsfrei |
| 47 | 2,2-Dimethyl-1,3-Propandiol | $HOCH_2C(CH_3)_2CH_2OH$ | 1,34 | 2,67 | 0,10 | 27,3 | 22,9 | 157 | 90,9 | -0,7 | 5,3 | schwach fruchtig |
| 48 | Neopentyl Glycol Propoxylat Polyol NS 20 (n=2) | $(CH_3)_2C[CH_2[OCH_2CH(CH_3)]_nOH]_2$ | 1,34 | 2,67 | 0,10 | 27,9 | 23,0 | 118 | 90,5 | -0,7 | 5,4 | schwach fruchtig |
| 49 | Trimethylolpropan Ethoxylat Polyol TP 08 (n=0,8) | $C_2H_5C[CH_2(OCH_2CH_2)_nOH]_3$ | 1,34 | 2,67 | 0,10 | 26,8 | 22,2 | 133 | 90,6 | -0,7 | 5,4 | geruchsfrei |
| 50 | Trimethylolpropan Ethoxylat Polyol TP 30 (n=3) | $C_2H_5C[CH_2(OCH_2CH_2)_nOH]_3$ | 1,34 | 2,67 | 0,10 | 27,2 | 23,6 | 83 | 89,8 | -0,7 | 5,7 | sehr schwach alkoholisch |

(fortgesetzt)

| Bsp. | Nachvernetzer oder Cosolvens | Formel | Nachvernetzer Gew.% | Wasser Gew.% | 2-Oxazolidon Gew.% | CRC (g/g) | AUL 0.7 psi (g/g) | SFC ($x10^{-7}cm^3xs/g$) | CIE-Farbzahl L | a | b | Geruchstest |
|---|---|---|---|---|---|---|---|---|---|---|---|---|
| 51 | Trimethylolpropan Ethoxylat Polyol TP 70 (n=7) | $C_2H_5C[CH_2(OCH_2CH_2)_nOH]_3$ | 1,34 | 2,67 | 0,10 | 27,7 | 24,6 | 59 | 89,4 | -0.7 | 5,8 | geruchsfrei |
| 52 | Trimethylolpropan Propoxylat Polyol TS 30 (n=3) | $C_2H_5C(CH_2(OC_3H_6)_nOH]_3$ | 1,34 | 2,67 | 0,10 | 28,1 | 24,0 | 67 | 90,2 | 0,7 | 5,7 | geruchsfrei |
| 53 | 2-Methyl-1,3-Propandiol | $HOCH_2CH(CH_3)CH_2OH$ | 1,34 | 2,67 | 0,10 | 26,4 | 22,8 | 164 | 90,5 | -0,7 | 5,3 | geruchsfrei |
| 54 | 3-Ethyl-3-Oxetanmethanol | 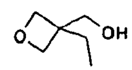 | 1,34 | 2,67 | 0,10 | 27,2 | 22,1 | 170 | 90,3 | -0,7 | 5,1 | geruchsfrei |
| 55 | 5-Ethyl-1,3-dioxan-5-methanol | 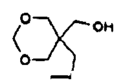 | 1.34 | 2,67 | 0,10 | 27,8 | 23,3 | 126 | 90,4 | -0,7 | 5,3 | geruchsfrei |
| 56 | Di-(Trimethylolpropan) | $O[CH_2C(C_2H_5)(CH_2OH)_2]_2$ | 1,34 | 2,67 | 0,10 | 27,6 | 23,7 | 59 | 89,8 | -0,8 | 6,1 | geruchsfrei |
| 57 | 1,1,1-Tris (Hydroxymethyl)propan | $C_2H_5C(CH_2OH)_3$ | 1,34 | 2,67 | 0.10 | 26,5 | 22,2 | 185 | 90,6 | -0,7 | 5,2 | geruchsfrei |

In den Beispielen 37, 38, 40 und 41 wurden wenig reaktive Nachvernetzer als Cosolventien eingesetzt, die stark riechende Nebenprodukte bilden, die einen Einsatz solchermaßen hergestellter wasserabsorbierender Polymerpartikel in Hygieneartikeln erschweren. Weiterhin neigen die Diole der Beispiele 37 und 38 zur Ausbildung einer leichten Gelbfärbung, ersichtlich am etwas erhöhten b-Farbzahlwert.

Die wasserabsorbierenden Polymerpartikel aus Beispiel 39 enthalten zwar keine unangenehm riechenden Verbindungen, jedoch hat sich hier ein zyklisches Nebenprodukt (Tetrahydrofuran) gebildet, das ebenfalls in Hygieneartikeln unerwünscht ist.

In den Beispielen 42 bis 57 wurden bevorzugte wenig reaktive Nachvernetzer als Colsoventien für reaktive Vernetzter, hier 2-Oxazolidon, verwendet. Diese Gemische führen zu praktisch geruchsfreien und weißen Produkten.

Beispiel 58 - Grundpolymer

In einem zweiarmigen Technikumskneter mit einem Arbeitsvolumen von 2 t wurden 1326 kg teilneutralisierte wässrige Natriumacrylatlösung mit einem Feststoffgehalt von 36 Gew.-% vorgelegt. Als Feststoffgehalt wird die Summe aus Acrylsäure und Natriumacrylat im Verhältnis zur Gesamtreaktionslösung betrachtet. Der Neutralisationsgrad betrug 65 mol-%. Es wurden als Vernetzer 0,40 Gew.-% (bezogen auf Acrylsäuremonomer) 18-fach ethoxyliertes Trimethylolpropantriacrylat hinzugefügt, gut gemischt und anschließend mit Stickstoff inertisiert. Die Temperatur dieser Lösung betrug 19˚C.

Durch zügige Zugabe unter Rühren von Natriumpersulfat (1,27 kg gelöst in 7,2 kg Wasser) und Ascorbinsäure (18,6 g gelöst in 3,7 kg Wasser) wurde gestartet, die Polymerisation wurde dann 45 Minuten unter kräftigem Kneten und Kühlung der Reaktorwände so fortgeführt, dass die maximale Temperatur im Kneter unter 100 ˚C blieb und ein fein zerteiltes klumpenfreies Gel erzeugt wurde.

Dieses wurde über einen Bandtrockner getrocknet, anschließend auf einem Walzenstuhl gemahlen und schließlich abgesiebt. Das erhaltene Polymerpulver hatte folgende Eigenschaften:

CRC = 34,2 g/g
AUL 0.3 psi = 12,3 g/g
Extrahierbare 16h = 13 Gew.-%
Restmonomer Acrylsäure = 240 ppm
Restfeuchte = 0,5 Gew.-%
pH = 5,7 - 5,8

Partikelgrössenverteitung

| | |
|---|---|
| >850 µm | 0,1 Gew.-% |
| 600-850 µm | 26,1 Gew.-% |
| 300-600 µm | 48,3 Gew.-% |
| 150-300 µm | 24,9 Gew.-% |
| 45-150 µm | 0,7 Gew.-% |
| <45 µm | <0,1 Gew.-% |
| | |
| 150-850 µm | 99,2 Gew.-% |

Beispiel 59 - Grundpolymer

Dieses Grundpolymer wurde vollkommen analog zu Beispiel 58 hergestellt, lediglich die Absiebung wurde entsprechend so geändert, dass ein geringerer Feinanteil im Produkt erzeugt wurde.

CRC =34,1 g/g
AUL 0.3 psi = 13,9 g/g
Extrahierbare 16h = 13 Gew.-%
Restmonomer Acrylsäure = 240 ppm
Restfeuchte = 0,5 Gew.-%

pH = 5,7

Partikelgrössenverteilung

| | |
|---|---|
| >850 µm | 0,1 Gew.-% |
| 600-850 µm | 29,9 Gew.-% |
| 300-600 µm | 55,1 Gew.-% |
| 150-300 µm | 14,6 Gew.-% |
| 45-150 µm | 0,4 Gew.-% |
| <45 µm | <0,1 Gew.-% |
| 150-850 µm | 99,6 Gew.-% |

Beispiel 60 - Grundpolymer

Dieses Grundpolymer wurde vollkommen analog zu Beispiel 58 hergestellt, lediglich die Absiebung wurde entsprechend so geändert, dass nur noch ein geringer Grobanteil im Produkt verblieb.

CRC = 33,9 g/g
AUL 0.3 psi = 11,6 g/g
Extrahierbare 16h = 12,9 Gew.-%
Restmonomer Acrylsäure = 230 ppm
Restfeuchte = 0,5 Gew.-%
pH = 5,7

Partikelgrössenverteilung

| | |
|---|---|
| >850 µm | <0,1 Gew.-% |
| 600-850 µm | 1,3 Gew.-% |
| 300-600 µm | 70,4 Gew.-% |
| 150-300 µm | 28,9 Gew.-% |
| 45-150 µm | 0,6 Gew.-% |
| <45 µm | <0,1 Gew.-% |
| 150-500 µm | 98,1 Gew.-% |

Beispiele 61 bis 66

In einem Lödige-Pflugscharkneter Typ VT 5R-MK (5 l Volumen) wurden jeweils 1,2 kg Grundpolymer aus Beispiel 60 vorgelegt. Dann wurde unter Verwendung des Lösungsmittelgemisches Isopropanol/Wasser in unten den tabellierten Mengen das jeweilige Vernetzergemisch gelöst in diesem Lösemittel mittels einer Zweistoffdüse unter Rühren aufgesprüht. Alle Mengenangaben in der Tabelle sind Gew.-%, bezogen auf vorgelegtes Grundpolymer. Gegebenenfalls wurde noch ein Additiv aufgebracht welches vorher in der Nachvernetzungslösung dispergiert oder gelöst wurde. Nach dem Aufsprühen wurde unter Rühren der Reaktormantel mittels Heizflüssigkeit aufgeheizt, wobei eine schnelle Aufheizrate vorteilhaft für die Produkteigenschaften ist. Die Heizung wurde so nachgeführt, dass das Produkt möglichst schnell die Zieltemperatur erreichte und dann dort stabil und unter Rühren getempert wurde. Regelmäßig wurden dann zu den in der Tabelle angegebenen Zeiten (nach Beginn des Aufheizens) Proben entnommen und die Eigenschaften bestimmt. Die Ergebnisse sind in den Tabellen 7 und 8 zusammengefasst.

Tab. 7: Ergebnisse der wasserabsorbierenden Polymerpartikel A (SFC, AUL und CRC)

| Bsp. | Lösemittel für Nachvernetzung [Gew.% bez. auf Polymer] auf | Nachvernetzer [Gew.% bezogen auf Polymer] | Additiv in der Oberflächen-nachvernetzungslösung [Gew.% bez. auf Polymer] | Öltemperatur im Heizmantel des Lödigereaktors (Anfang/Ende) [°C] | Produkt-temperatur | Zeit | SFC [$10^{-7}cm^3$ s $g^{-1}$] | AUL | CRC |
|---|---|---|---|---|---|---|---|---|---|
| | | | | | [°C] | [min] | | 0.7 psi | |
| | | | | | | | | (g/g) | (g/g) |
| 61 | 1.2% Isopropanol 2.8% Wasser | 0.1% HEONON 0.1% 1,3-Propandiol | | 215 - 202 | 175 | 60 | 33 | 24,6 | 28,7 |
| | | | | | 175 | 70 | 48 | 24,3 | 27,8 |
| | | | | | 175 | 80 | 57 | 23,8 | 27,6 |
| 62 | 1.2 % Isopropanol 2.8% Wasser | 0.1% HEONON 0.1% 1,3-Propandiol | | 250 - 218 | 185 | 30 | 42 | 25,1 | 28,1 |
| | | | | | 185 | 45 | 88 | 23,3 | 24,9 |
| | | | | | 185 | 80 | 128 | 21,6 | 23,3 |
| 63 | 1.2 % Isopropanol 2.8 % Wasser | 0.1% HEONON 0.1% 1,3-Propandiol | | 240 - 203 | 175 | 45 | 33 | 25,5 | 29,0 |
| | | | | | 175 | 60 | 57 | 25,0 | 27,0 |
| | | | | | 175 | 70 | 64 | 24,5 | 26,5 |
| | | | | | 175 | 80 | 80 | 24,1 | 26,3 |
| 64 | 1.2 % Isopropanol 64 2.8% Wasser | 0.1% HEONON 0.1% 1,3-Propandiol | 0.5% Calciumphosphat (C13-09, Fa. Budenheim) | 240 - 204 | 175 | 45 | 60 | 25,8 | 28,8 |
| | | | | | 175 | 60 | 91 | 25,0 | 27,0 |
| | | | | | 175 | 70 | 107 | 24,3 | 26,3 |
| | | | | | 175 | 80 | 108 | 23,8 | 25,5 |
| 65 | 1.2% Isopropanol 2.8 % Wasser | 0.1% HEONON 0.1% 1,3-Propandiol | 0.25% Aluminiumsulfat | 240 - 204 | 175 | 45 | 48 | 23,8 | 27,9 |
| | | | | | 175 | 60 | 73 | 24,2 | 27,7 |
| | | | | | 175 | 70 | 85 | 23,2 | 26,4 |
| | | | | | 175 | 80 | 94 | 22,3 | 26,3 |

(fortgesetzt)

| Bsp. | Lösemittel für Nachvernetzung [Gew.% bez. auf Polymer] auf | Nachvernetzer [Gew.% bezogen auf Polymer] | Additiv in der Oberflächen-nachvernetzungslösung [Gew.% bez. auf Polymer] | Öltemperatur im Heizmantel des Lödigereaktors (Anfang/Ende) [°C] | Produkt-temperatur | Zeit | SFC [$10^{-7} cm^3 s g^{-1}$] | AUL | CRC |
|---|---|---|---|---|---|---|---|---|---|
| | | | | | [°C] | [min] | | 0.7 psi | |
| | | | | | | | | (g/g) | (g/g) |
| 66 | 1.2 % Isopropanol<br>2.8 % Wasser | 0.1% HEONON<br>0.1% 1,3-Propandiol | 0.5% Calciumphosphat (C13-09,Fa. Budenheim)<br>0.2% Boltorn H40,(Fa. Perstorp) | 240 - 204 | 175 | 45 | 63 | 25,6 | 29,5 |
| | | | | | 175 | 60 | 116 | 25,2 | 27,9 |
| | | | | | 175 | 70 | 119 | 23,7 | 27.3 |
| | | | | | 175 | 80 | 128 | 23,8 | 26,4 |

Tab. 8: Ergebnisse der wasserabsorbierenden Polymerpartikel A ($DA_{60}$, $DA_{240}$ und TW)

| Beispiel | Zeit | | Dochtwirkungstest | TW | |
|---|---|---|---|---|---|
| | [min] | 60 min | 240 min | SFC x $DA_{60}$ x $10^7$ | SFC x $DA_{240}$ x $10^7$ |
| | | $DA_{60}$ | $DA_{240}$ | | |
| | | (g/70g) | (g/70g) | | |
| 61 | 60 | | | | |
| | 70 | | | | |
| | 80 | **303** | **412** | **17389** | **23609** |
| 62 | 30 | | | | |
| | 45 | **354** | **507** | **31150** | **44605** |
| | 80 | **397** | **521** | **50626** | **66454** |
| 63 | 45 | | | | |
| | 60 | | | | |
| | 70 | | | | |
| | 80 | **370** | **499** | **29633** | **39884** |
| 64 | 45 | | | | |
| | 60 | | | | |
| | 70 | | | | |
| | 80 | **264** | **401** | **28464** | **43257** |
| 65 | 45 | | | | |
| | 60 | | | | |
| | 70 | | | | |
| | 80 | **234** | **361** | **22015** | **33952** |
| 66 | 45 | | | | |
| | 60 | | | | |
| | 70 | | | | |
| | 80 | **192** | **300** | **24600** | **38540** |

Beispiel 67 bis 78

Vollkommen analog zu den Beispielen 61 bis 66 wurden die Beispiele 67 bis 78 durchgeführt, jedoch wurden hier die Grundpolymere 58 und 59 eingesetzt.

Tab. 9: Ergebnisse der wasserabsorbierenden Polymerpartikel B und C (SFC, AUL, CRC, FSR, $DA_{60}$, $DA_{240}$ und TW)

| Bsp. | Grund-polymer aus Beispiel | Lösemittel | Nachvernetzer | Temp. | Zeit | SFC | AUL | CRC | FSR | Dochtwirkungstest | | TW |
|---|---|---|---|---|---|---|---|---|---|---|---|---|
| | | | | ˚C | Min. | x $10^{-7} cm^3 s g^{-1}$ | 0.7 psi | | | 60 min | 240 min | SFC x SFC x $10^7$ x $DA_{60}$ |
| | | | | | | | (g/g) | (g/g) | (g/gs) | (g/70g) | (g/70g) | |
| 67 | **58** | 1.2% Isopropanol 2.8% Wasser | 0.1% 2-Oxazolidinon 0.1% 1.3-Propandiol | 175 | 30 | 30 | 25,5 | 28,3 | | | | |
| | | | | 175 | 45 | 56 | 25,0 | 28,4 | 0.22 | 285 | 375 | 16048 |
| | | | | 175 | 60 | 62 | 23,9 | 27,5 | | | | |
| 68 | **58** | 1.2% Isopropanol 2.8% Wasser | 0.1% HEONON 0.1% 1,3-Propandiol | 175 | 60 | 51 | 25,4 | 29,6 | 0,19 | | | |
| 69 | **58** | 1.2% Isopropanol 2.8% Wasser | 0.1% EGDGE 0.1% 1,3-Propandiol | 175 | 20 | 34 | 25,4 | 28,7 | | | | |
| | | | | 175 | 30 | 42 | 25,6 | 27,8 | | | | |
| | | | | 175 | 45 | 53 | 24,9 | 28,0 | 0,18 | 295 | 395 | 15629 |
| | | | | 175 | 60 | 58 | 24,3 | 27,6 | | | | |
| 70 | **58** | 1.2% Isopropanol 2.8% Wasser | 0.1% HEONON | 175 | 70 | 27 | 25,6 | 29,7 | | | | |
| | | | | 175 | 80 | 30 | 25,7 | 29,1 | | | | |
| 71 | **58** | 1.2%Isopropanol 2.8% Wasser | 0.1% 1,3-Propandiol | 175 | 70 | 26 | 25,2 | 30,0 | | | | |
| | | | | 175 | 80 | 35 | 25,0 | 29,0 | | | | |
| 72 | **58** | 1.2% Isopropanol 2.8% Wasser | 0.1% HEONON 0.1% 1,3-Propandiol 0.5% SEA (C13-09) | 175 | 60 | 40 | 25,4 | 30,2 | | | | |
| | | | | 175 | 70 | 67 | 25,2 | 29,5 | | | | |
| | | | | 175 | 80 | 89 | 24,0 | 28,1 | | 171 | 327 | 15130 |
| 73 | **59** | 1.2% Isopropanol 2.8% Wasser | 0.1% HEONON 0.1% 1,3-Propandiol | 175 | 45 | 48 | 26,0 | 30,0 | 0,17 | | | |
| | | | | 175 | 60 | 79 | 25,7 | 28,6 | | | | |
| | | | | 175 | 70 | 89 | 25,2 | 27,7 | | | | |
| | | | | 175 | 80 | 108 | 24,9 | 26,8 | | | | |

(fortgesetzt)

| Bsp. | Grund-polymer aus Beispiel | Lösemittel | Nachvernetzer | Temp. | Zeit | SFC | AUL | CRC | FSR | Dochtwirkungstest | | TW |
|---|---|---|---|---|---|---|---|---|---|---|---|---|
| | | | | ˚C | Min. | x $10^{-7} cm^3 s g^{-1}$ | 0.7 psi | | | 60 min | 240 min | SFC x SFC x $10^7$x $DA_{60}$ |
| | | | | | | | (g/g) | (g/g) | (g/gs) | (g/70g) | (g/70g) | |
| 74 | **59** | 1.2% Isopropanol 2.8% Wasser | 0.1% HEONON | 175 | 60 | 30 | 24,9 | 31,2 | | | | |
| | | | | 175 | 70 | 43 | 25,0 | 30,5 | 0,16 | | | |
| | | | | 175 | 80 | 60 | 25,3 | 29,2 | | | | |
| 75 | **59** | 1.2% Isopropanol 2.8% Wasser | 0.1%1,3-Propandiol | 175 | 60 | 32 | 24,7 | 30,6 | | | | |
| | | | | 175 | 70 | 45 | 25,2 | 29,6 | 0,17 | | | |
| | | | | 175 | 80 | 64 | 25,1 | 29,0 | | | | |
| 76 | **59** | 1.2% Isopropanol 2.8% Wasser | 0.12% HEONON 0.08% 1,2-Propandiol | 175 | 45 | 26 | 24,6 | 31,3 | | | | |
| | | | | 175 | 60 | 53 | 25,6 | 29,9 | 0,19 | | | |
| 77 | **59** | 1.2% Isopropanol 2.8% Wasser | 0.2% 1,3-Propandiol | 175 | 60 | 41 | 26,0 | 29,6 | | | | |
| | | | | 175 | 70 | 59 | 25,7 | 28,9 | 0,16 | | | |
| | | | | 175 | 80 | 82 | 25,1 | 28,3 | | 259 | 347 | 21305 |
| 78 | **59** | 1.2% Isopropanol 2.8% Wasser | 0.1 % HEONON 0.1% 1,3-Propandiol 0.5% SEA (C13-09) | 175 | 45 | 36 | 25,4 | 30,5 | | | | |
| | | | | 175 | 60 | 77 | 24,8 | 28,6 | | | | |
| | | | | 175 | 80 | 89 | 23,9 | 27,3 | | 212 | 389 | 18836 |

## Patentansprüche

1. Wasserabsorbierende Polymerpartikel, enthaltend

   a) mindestens ein einpolymerisiertes ethylenisch ungesättigtes, säuregruppentragendes Monomer,
   b) mindestens einen einpolymerisierten Vernetzer,
   c) gegebenenfalls ein oder mehrere mit a) copolymerisierbare einpolymerisierte ethylenisch und/oder allylisch ungesättigte Monomere,
   d) gegebenenfalls ein oder mehrere wasserlösliche Polymere, auf die die Monomere a), b) und ggf. c) zumindest teilweise aufgepfropft sind, und
   e) mindestens einen umgesetzten Nachvernetzer,

   wobei die Polymerpartikel mindestens ein wasserunlösliches Metallphosphat enthalten, mindestens 90 Gew.-% der Polymerpartikel eine Partikelgröße von 150 bis 850 μm und mindestens 16 Gew.-% der Polymerpartikel eine Partikelgröße von weniger als 300 μm aufweisen, die Zentrifugenretentionskapazität (CRC) mindestens 26 g/g und der Transportwert (TW) mindestens 15.000 $cm^3s$ beträgt,
   wobei der Transportwert (TW) das Produkt aus Flüssigkeitsweiterleitung (SFC) und Dochtabsorption nach 60 Minuten ($DA_{60}$) multipliziert mit $10^7$ ist, und
   wobei die Dochtabsorption nach 60 Minuten ($DA_{60}$) die Gewichtsmenge an 0,9 gew.-%iger Kochsalzlösung ist, die 70 g der wasserabsorbierenden Polymerpartikel in 60 Minuten aufnehmen, wobei sich die wasserabsorbierenden Polymerpartikel während der Messung in einem kreisrunden Gefäß mit einem Innendurchmesser von 6 cm befinden, welches unten mit einen Siebboden der Maschenweite 36 μm verschlossen ist, und der Siebboden drucklos mit 0,9 gew.-%iger Kochsalzlösung in Kontakt steht.

2. Polymerpartikel gemäß Anspruch 1, wobei weniger als 2 Gew.-% der Polymerpartikel eine Partikelgröße von weniger als 150 μm aufweisen.

3. Polymerpartikel gemäß Anspruch 1 oder 2, wobei die Säuregruppen des mindestens einen säuregruppentragenden Monomeren zu größer 60 und höchstens 70 mol-% neutralisiert sind.

4. Polymerpartikel gemäß einem der Ansprüche 1 bis 3, wobei weniger als 2 Gew.-% der Polymerpartikel eine Partikelgröße von über 850 μm aufweisen.

5. Polymerpartikel gemäß einem der Ansprüche 1 bis 4, wobei die Polymerpartikel eine Flüssigkeitsweiterleitung (SFC) von mindestens $45 \times 10^{-7} cm^3s/g$ aufweisen.

6. Polymerpartikel gemäß einem der Ansprüche 1 bis 5, wobei die Polymerpartikel eine Zentrifugenretentionskapazität (CRC) von mindestens 30 g/g aufweisen.

7. Verfahren zur Herstellung wasserabsorbierender Polymerpartikel, durch Polymerisation einer Monomerlösung, enthaltend

   i) mindestens ein ethylenisch ungesättigtes, säuregruppentragendes Monomer,
   ii) mindestens einen Vernetzer,
   iii) gegebenenfalls ein oder mehrere mit i) copolymerisierbare ethylenisch und/oder allylisch ungesättigte Monomere und
   iv) gegebenenfalls ein oder mehrere wasserlösliche Polymere, auf die die Monomere i), ii) und ggf. iii) zumindests teilweise aufgepfropft werden können,
   wobei das dabei erhaltene Grundpolymer getrocknet, klassiert, mit
   v) mindestens einem Nachvernetzer,
   nachbehandelt, getrocknet und thermisch nachvernetzt wird, **dadurch gekennzeichnet, dass** die thermische Nachvernetzung abgebrochen wird nachdem die wasserabsorbierenden Polymerpartikel eine Zentrifugenretentionskapazität (CRC) von mindestens 26 g/g und einen Transportwert (TW) von mindestens 15.000 $cm^3s$ aufweisen,
   wobei der Transportwert (TW) das Produkt aus Flüssigkeitsweiterleitung (SFC) und Dochtabsorption nach 60 Minuten ($DA_{60}$) multipliziert mit $10^7$ ist, und
   wobei die Dochtabsorption nach 60 Minuten ($DA_{60}$) die Gewichtsmenge an 0,9 gew.-%iger Kochsalzlösung ist, die 70 g der wasserabsorbierenden Polymerpartikel in 60 Minuten aufnehmen, wobei sich die wasserabsorbierenden Polymerpartikel während der Messung in einem kreisrunden Gefäß mit einem Innendurchmesser von 6 cm befinden, welches unten mit einen Siebboden der Maschenweite 36 µm verschlossen ist, und der Siebboden drucklos mit 0,9 gew.-%iger Kochsalzlösung in Kontakt steht
und wobei die Polymerpartikel mit mindestens einem wasserunlöslichen Metallphosphat beschichtet werden, sowie mindestens 90 Gew.-% der Polymerpartikel eine Partikelgröße von 150 bis 850 µm und mindestens 16 Gew.-% der Polymerpartikel eine Partikelgröße von weniger als 300 µm aufweisen.

**8.** Verfahren gemäß Anspruch 7, **dadurch gekennzeichnet, dass** die Säuregruppen des mindestens einen säuregruppentragenden Monomeren im Grundpolymer zu größer 60 und höchstens 70 mol-% neutralisiert sind.

**9.** Verfahren gemäß Anspruch 7 oder 8, **dadurch gekennzeichnet, dass** der mindestens eine Nachvernetzer ein Amidacetal, ein Carbaminsäureester, ein zyklischer Kohlensäureester, ein Bisoxazolin und/oder ein mehrwertiger Alkohol ist, wobei der mehrwertige Alkohol ein Molekulargewicht von weniger als 100 g/mol pro Hydroxylgruppe sowie keine vincinalen, geminalen, sekundären oder tertiären Hydroxylgruppen aufweist.

**10.** Verfahren gemäß einem der Ansprüche 7 bis 9, **dadurch gekennzeichnet, dass** die Nachvernetzung bei einer Temperatur von 160 bis 210˚C durchgeführt wird.

**11.** Verfahren gemäß einem der Ansprüche 7 bis 10, **dadurch gekennzeichnet, dass** der mindestens eine Nachvernetzer einen Siedepunkt von höchstens 160˚C oder mindestens 220˚C beträgt.

**12.** Verfahren gemäß einem der Ansprüche 7 bis 11, **dadurch gekennzeichnet, dass** der Nachvernetzer zusammen mit mindestens einem Cosolvens als wässrige Lösung eingesetzt wird, wobei der Siedepunkt des Cosolvens höchstens 160˚C oder mindestens 220˚C beträgt.

**13.** Verfahren gemäß einem der Ansprüche 7 bis 12, **dadurch gekennzeichnet, dass** der mindestens eine Nachvernetzer als wässrige Lösung in Abwesenheit eines Cosolvens eingesetzt wird.

**14.** Hygieneartikel, enthaltend wasserabsorbierende Polymerpartikel gemäß einem der Ansprüche 1 bis 6.

**15.** Hygieneartikel, enthaltend wasserabsorbierende Polymerpartikel hergestellt gemäß einem Verfahren gemäß einem der Ansprüche 7 bis 13.

FIG.1A

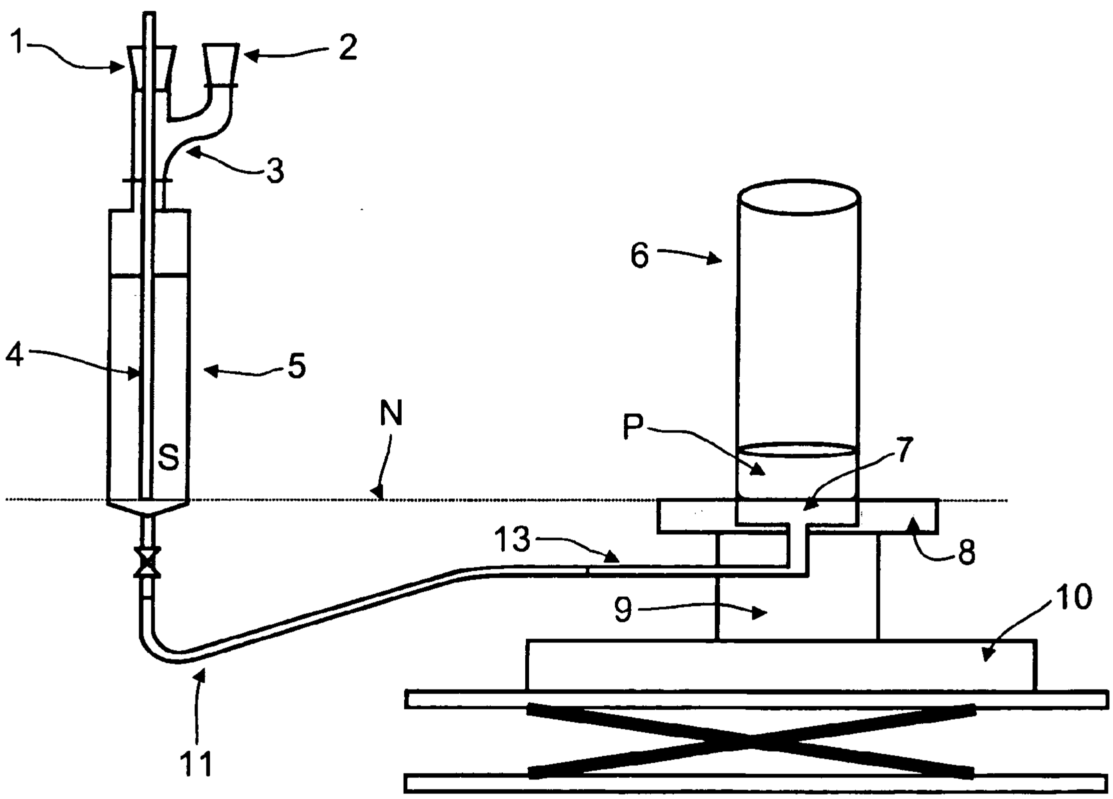

FIG.1B

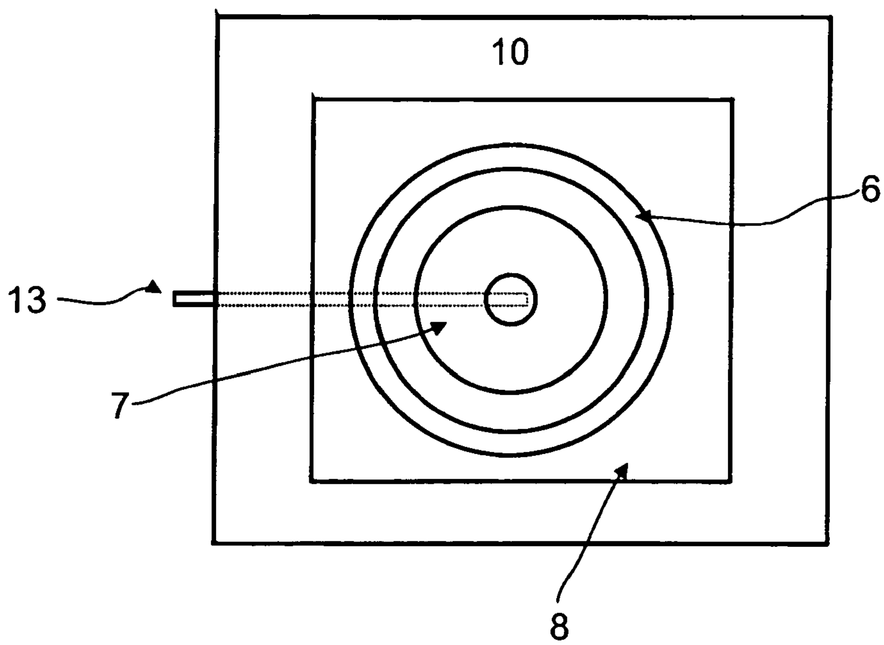

Europäisches Patentamt
European Patent Office
Office européen des brevets

# EUROPÄISCHER RECHERCHENBERICHT

Nummer der Anmeldung
EP 10 17 3417

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2004/052949 A (BASF AKTIENGESELLSCHAFT; NESTLER, GERHARD; MUELLER-ENGEL, KLAUS JOACHI) 24. Juni 2004 (2004-06-24)<br>* das ganze Dokument *<br>----- | 1-15 | INV.<br>A61L15/60<br>A61L15/24<br>A61L15/18 |
| X,D | WO 02/060983 A (BASF AKTIENGESELLSCHAFT; DANIEL, THOMAS; RIEGEL, ULRICH; WEISMANTEL, M) 8. August 2002 (2002-08-08)<br>* das ganze Dokument *<br>----- | 1-15 | |
| A | WO 2004/024816 A (BASF AKTIENGESELLSCHAFT; DANIEL, THOMAS; RIEGEL, ULRICH; ELLIOTT, MARK) 25. März 2004 (2004-03-25)<br>* das ganze Dokument *<br>----- | 1-15 | |
| A | WO 99/42494 A1 (BASF AG [DE]; FUNK RUEDIGER [DE]; FRENZ VOLKER [DE]; RIEGEL ULRICH [DE) 26. August 1999 (1999-08-26)<br>* das ganze Dokument *<br>----- | 1-15 | |
| | | | RECHERCHIERTE SACHGEBIETE (IPC) |
| A,P | WO 2005/080479 A (BASF AKTIENGESELLSCHAFT; RIEGEL, ULRICH; DANIEL, THOMAS; WEISMANTEL, M)<br>1. September 2005 (2005-09-01)<br>* das ganze Dokument *<br>----- | 1-15 | A61L |
| E | WO 2005/097881 A (BASF AKTIENGESELLSCHAFT; RIEGEL, ULRICH; DANIEL, THOMAS; WEISMANTEL, M)<br>20. Oktober 2005 (2005-10-20)<br>* das ganze Dokument *<br>----- | 1-15 | |
| E | WO 2006/015729 A (BASF AKTIENGESELLSCHAFT; EXNER, KAI MICHAEL; DANIEL, THOMAS; ELLIOTT,) 16. Februar 2006 (2006-02-16)<br>* Beispiele 7-14 *<br>----- | 1-15 | |

Der vorliegende Recherchenbericht wurde für alle Patentansprüche erstellt

1

| Recherchenort | Abschlußdatum der Recherche | Prüfer |
|---|---|---|
| München | 26. Oktober 2010 | Hars, Jesko |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 10 17 3417

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-10-2010

| Im Recherchenbericht angeführtes Patentdokument | Datum der Veröffentlichung | Mitglied(er) der Patentfamilie | Datum der Veröffentlichung |
|---|---|---|---|
| WO 2004052949 A | 24-06-2004 | AT 329943 T | 15-07-2006 |
| | | AU 2003294814 A1 | 30-06-2004 |
| | | BR 0316864 A | 18-10-2005 |
| | | CA 2507548 A1 | 24-06-2004 |
| | | CN 1723223 A | 18-01-2006 |
| | | DE 10257449 A1 | 06-11-2003 |
| | | EP 1583779 A1 | 12-10-2005 |
| | | JP 2006509085 T | 16-03-2006 |
| | | KR 20050089813 A | 08-09-2005 |
| | | MX PA05005775 A | 16-08-2005 |
| | | US 2006036043 A1 | 16-02-2006 |
| | | ZA 200505518 A | 25-10-2006 |
| WO 02060983 A | 08-08-2002 | AU 2002250836 A1 | 12-08-2002 |
| | | CN 1501957 A | 02-06-2004 |
| | | EP 1366111 A2 | 03-12-2003 |
| | | JP 2004517728 T | 17-06-2004 |
| | | US 2004077796 A1 | 22-04-2004 |
| WO 2004024816 A | 25-03-2004 | AT 336548 T | 15-09-2006 |
| | | AU 2003264101 A1 | 30-04-2004 |
| | | BR 0313757 A | 21-06-2005 |
| | | CA 2496448 A1 | 25-03-2004 |
| | | CN 1678681 A | 05-10-2005 |
| | | DE 10239074 A1 | 11-03-2004 |
| | | EP 1537177 A1 | 08-06-2005 |
| | | ES 2271690 T3 | 16-04-2007 |
| | | JP 4278613 B2 | 17-06-2009 |
| | | JP 2005537131 T | 08-12-2005 |
| | | KR 20050038033 A | 25-04-2005 |
| | | MX PA05001851 A | 03-06-2005 |
| | | US 2005245684 A1 | 03-11-2005 |
| | | ZA 200502432 A | 04-10-2005 |
| WO 9942494 A1 | 26-08-1999 | AU 2927199 A | 06-09-1999 |
| | | BR 9908006 A | 24-10-2000 |
| | | CA 2320778 A1 | 26-08-1999 |
| | | CN 1291202 A | 11-04-2001 |
| | | DE 19807502 A1 | 16-09-1999 |
| | | EP 1056787 A1 | 06-12-2000 |
| | | ES 2205788 T3 | 01-05-2004 |
| | | ID 25873 A | 09-11-2000 |
| | | JP 4210432 B2 | 21-01-2009 |
| | | JP 2002504566 T | 12-02-2002 |
| | | US 6472478 B1 | 29-10-2002 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR.

EP 10 17 3417

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-10-2010

| Im Recherchenbericht angeführtes Patentdokument | Datum der Veröffentlichung | Mitglied(er) der Patentfamilie | Datum der Veröffentlichung |
|---|---|---|---|
| WO 2005080479 A | 01-09-2005 | BR PI0507793 A | 17-07-2007 |
| | | CN 1922243 A | 28-02-2007 |
| | | DE 102004009438 A1 | 15-09-2005 |
| | | EP 1720934 A1 | 15-11-2006 |
| | | JP 4395531 B2 | 13-01-2010 |
| | | JP 2007523254 T | 16-08-2007 |
| | | KR 20070032299 A | 21-03-2007 |
| | | US 2007161759 A1 | 12-07-2007 |
| | | ZA 200607943 A | 25-06-2008 |
| WO 2005097881 A | 20-10-2005 | CN 1938371 A | 28-03-2007 |
| | | DE 102004015686 A1 | 27-10-2005 |
| | | EP 1735375 A1 | 27-12-2006 |
| | | JP 2007530752 T | 01-11-2007 |
| | | US 2007244283 A1 | 18-10-2007 |
| WO 2006015729 A | 16-02-2006 | CN 1993150 A | 04-07-2007 |
| | | DE 102004038015 A1 | 16-03-2006 |
| | | EP 1776147 A2 | 25-04-2007 |
| | | JP 2008508407 T | 21-03-2008 |
| | | US 2008171837 A1 | 17-07-2008 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5599335 A
- WO 04069293 A
- WO 04069404 A
- WO 04069915 A
- EP 530438 A
- EP 547847 A
- EP 559476 A
- EP 632068 A
- WO 9321237 A
- WO 03104299 A
- WO 03104300 A
- WO 03104301 A
- DE 10331450
- DE 10331456
- DE 10355401
- DE 19543368 A
- DE 19646484 A
- WO 9015830 A
- WO 0232962 A
- EP 343427 A
- DE 10319462
- DE 19941423 A
- EP 686650 A
- WO 0145758 A
- WO 0138402 A
- EP 955086 A
- EP 083022 A
- EP 543303 A
- EP 937736 A
- DE 3314019 C
- DE 3523617 C
- EP 450922 A
- DE 10204938 A
- US 6239230 B
- EP 1199327 A
- DE 4020780 A
- DE 198075022 A
- DE 19807992 A
- DE 198545732 A
- DE 10204937 A
- DE 10334584
- WO 03031482 A
- EP 534228 A
- EP 1191051 A
- WO 02060983 A
- US 20030181115 A
- US 20040019342 A
- EP 377199 A
- EP 445641 A
- US 5026806 A
- EP 655465 A
- EP 377191 A
- EP 640330 A


### In der Beschreibung aufgeführte Nicht-Patentliteratur


- Hunterlab. Januar 1996, vol. 8, 1-4